(19) Europäisches Patentamt
European Patent Office
Office européen des brevets

(11) EP 3 331 859 B1

(12) **EUROPEAN PATENT SPECIFICATION**

(45) Date of publication and mention of the grant of the patent:
**02.10.2019 Bulletin 2019/40**

(21) Application number: **16747883.3**

(22) Date of filing: **29.07.2016**

(51) Int Cl.:
***C07D 213/74*** (2006.01) ***C07D 401/12*** (2006.01)
***A61K 31/4709*** (2006.01) ***A61P 25/00*** (2006.01)

(86) International application number:
**PCT/EP2016/068199**

(87) International publication number:
**WO 2017/021319 (09.02.2017 Gazette 2017/06)**

(54) **BROAD SPECTRUM REACTIVATORS OF OPNA-INHIBITION OF HUMAN CHOLINESTERASES**

BREITSPEKTRUMREAKTIVATOREN VON OPNA-INHIBITION VON MENSCHLICHEN CHOLINESTERASEN

RÉACTIVATEURS À LARGE SPECTRE DE L'INHIBITION DE L'OPNA DE CHOLINESTÉRASES HUMAINES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.08.2015 EP 15306257**

(43) Date of publication of application:
**13.06.2018 Bulletin 2018/24**

(73) Proprietors:
- **Centre National de la Recherche Scientifique**
  **75016 Paris (FR)**
- **Université de Strasbourg**
  **67000 Strasbourg (FR)**

(72) Inventors:
- **BAATI, Rachid**
  **67460 Souffelweyersheim (FR)**
- **BROWN, Richard, C., D.**
  **Southampton SO17 1BJ (GB)**
- **DIAS, José**
  **94230 Cachan (FR)**
- **NACHON, Florian**
  **38610 Gieres (FR)**
- **DE SOUSA, Julien**
  **77270 Villeparisis (FR)**

(74) Representative: **Cabinet Becker et Associés**
**25, rue Louis le Grand**
**75002 Paris (FR)**

(56) References cited:
**WO-A1-2015/075082**

- **KLIACHYNA M. ET AL.: EUROPEAN JOURNAL OF MEDICINAL CHEMISTRY, vol. 78, 2014, pages 455-467, XP028847852, cited in the application**
- **JULIEN RENOU ET AL: "Syntheses and in vitro evaluations of uncharged reactivators for human acetylcholinesterase inhibited by organophosphorus nerve agents", CHEMICO-BIOLOGICAL INTERACTIONS., vol. 203, no. 1, 1 March 2013 (2013-03-01) , pages 81-84, XP055220523, IR ISSN: 0009-2797, DOI: 10.1016/j.cbi.2012.09.023**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Introduction

The present invention deals with novel reactivators of human acetylcholinesterase (hAChE), pharmaceutical compositions comprising said compounds, and their use for reactivating human acetylcholinesterase inhibited by at least one organophosphorous nerve agent.

### Background of the invention

Organophosphorous nerve agents (OPNA) are extremely toxic compounds that comprise chemical warfare agents (CWA) including sarin, soman, cyclosarin, tabun, methylphosphonothioate (VX) and pesticides such as paraoxon, parathion and tetraethyl pyrophosphate (TEPP). Their acute toxicity results from the irreversible inhibition of acetylcholinesterase (AChE) through phosphylation of its catalytic serine, which results in the inability of the enzyme to hydrolyze acetylcholine (ACh). Accumulation of this neurotransmitter at cholinergic synapses occurs, leading to a permanent saturation of the muscarinic and nicotinic receptors which ultimately results in seizure and respiratory arrest. Depending on the class of OPNA and on the administrated dose, death can occur within a few minutes.
Due to the similarity between the chemical precursors of CWA and pesticides, and to the relatively simple chemistry involved in their synthesis, efforts to control the proliferation of these agents have proved of limited success. Illustrative examples include the terrorist attack in the Tokyo subway in 1995, the bombing of Kurd civilians during the Iraq-Iran war in 1988, and that of civilians in Syria, as reported in August 2013. Additionally, despite the international efforts aimed at regulating and lessening the use of these environmentally toxic compounds, ca. 100 different OPNA are still used intensively as pest control agents, with only anecdotal monitoring. This results in about 3,000,000 acute intoxications per year, 200,000 of which lead to death. Moreover, intoxications may also occur during the destruction of chemical weapons stockpiles.
Therefore, the development of effective measures to counteract OPNA poisoning remains a challenging issue to protect and treat both civilian and military populations. The current treatment for OPNA poisoning consists of the administration of a combination of atropine (antimuscarinic agent) and diazepam (anticonvulsant drug), to limit convulsions, and of a standard pyridinium oxime (pralidoxime, trimedoxime, HI-6, obidoxime, or HLö-7) to reactivate AChE. Oximes exert their action on OPNA-inhibited AChE by attacking the phosphorous atom of the phosphylated serine, leading to the removal of the phosphonate and restoration of the enzyme's catalytic activity. Some of these known compounds have a pyridinium oxime-based structure coupled to a potential ligand for the peripheral site of the enzyme (PSL), the purpose of which is to increase the affinity of the reactivator for AChE (Mercey G. et al., Accounts of Chemical Research, 756-766, 2012, Vol. 45, No. 5). The reactivation process can be illustrated by the following scheme:

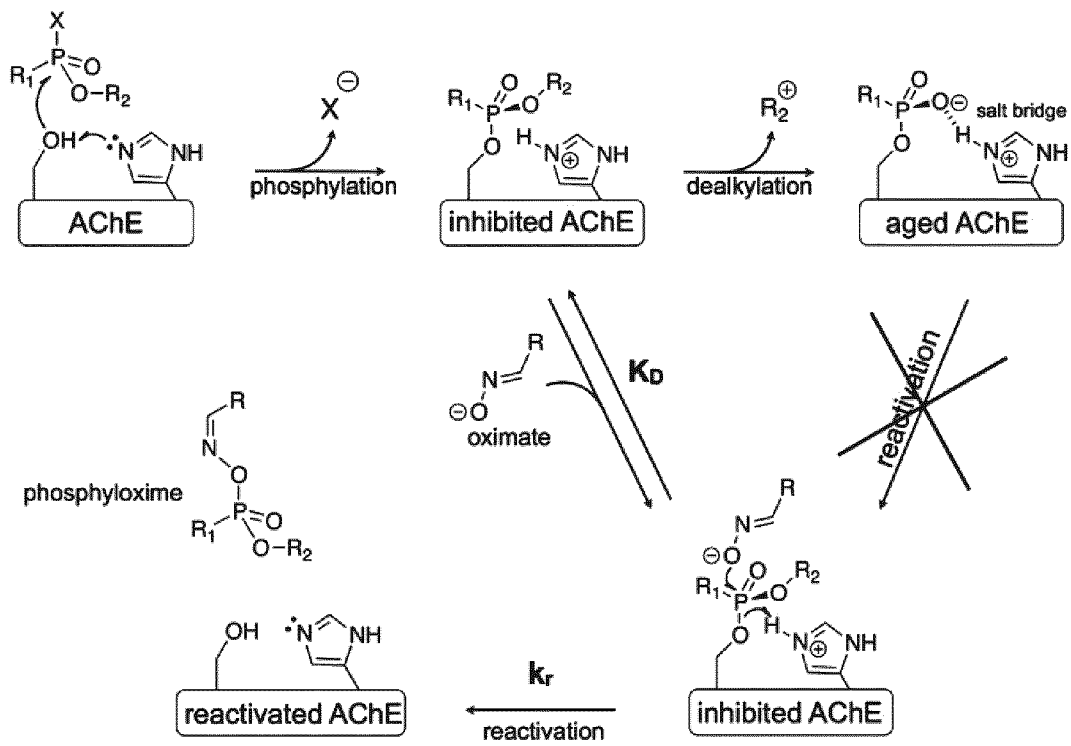


The efficiency of reactivators may be estimated by the second-order rate constant for reactivation $k_{r2}$, which is the ratio of the maximal reactivation rate constant ($k_r$) to the apparent dissociation constant of the reactivator - inhibited AChE complex ($K_D$).

As of today, none of the known oximes has proven equally effective against all types of OPNA-inhibited AChE, and most are ineffective against tabun-inhibited hAChE. This is because tabun-inhibited enzyme is resistant to reactivation due to weak electrophilicity and steric hindrance of the tabun-hAChE adduct.

Recently, the present inventors have synthetised bifunctional reactivators comprising a tetrahydroacridine (tacrine)-type peripheral site ligand bonded to an oxime moiety via a flexible linker (WO 2015/075082). These compounds have proven to be effective against hAChE inhibited by various OPNA and even more effective than trimedoxime against tabun-inhibited hAChE (Kliachyna M. et al., European Journal of Medicinal Chemistry, 78, 2014, 455-467). However, the reactivation efficiency of these compounds could still be improved.

## Summary of the invention

In this context, the Applicant found that bifunctional compounds comprising a specific peripheral site ligand (PSL) moiety of the amino-quinoline type had improved affinity for poisoned hAChE (thus, a lower $K_D$) than the tacrine-based reactivators, which allowed them to be more potent reactivators of human AChE inhibited with any type of organophosphorous compounds. These novel compounds may thus be used at a lower concentration, which decreases the possible side effects and cost of the treatment.

This result was clearly unprecedented for at least three reasons. First, amino-quinoline is not known by itself as an inhibitor of hAChE. Secondly, the novel structure appears to be simplified compared to the known tacrine-based structure and essentially amounts to the removal of the tacrine moiety. By contrast, the literature suggests that the reactivation kinetics could be controlled by structural modification of the PSL moiety (Saint-André et al., Tetrahedron, 67, 2011, 6352-6361) which, according to drug design practice, rather amounts to grafting specific groups to a known structure. Thirdly, the reactivation mechanism of OPNA-inhibited hAChE is still not fully understood, such that it is not easy to design compounds having a proper $k_r/K_D$ ratio. The key to the improvement of the reactivation potency is finding the optimal balance between affinity of the functional reactivator for the enzyme and the positioning of the oxime function relative to the phosphorous atom of the OPNA-hAChE adduct. In the design of reactivators, one of the numerous issues to address is that some reactivation products, which are phosphyloximes, may themselves display high affinity for the enzyme. Therefore, the design of potent reactivators for the medical treatment of OPNA poisoning with a broad activity spectrum was not straightforward.

The compounds of this invention allow for effective and fast reactivation of hAChE without denaturing the same. They are thus useful for treating people poisoned by organophosphorous compounds and especially civilian and military

people contaminated by warfare agents, farmers intoxicated by pest control agents and chemists who handle organophosphorous products. Moreover, these compounds may be produced under economically favorable conditions since they may be obtained using simple synthesis steps on a scale that may be easily adjusted.
Incidentally, these novel compounds have been shown to be able to inhibit native hAChE at a concentration range of micro- to nanomolar. They may thus be used in the treatment of various neurological diseases in which inhibitors of hAChE have proven to be useful, such as Alzheimer's disease.

A first object of the present invention is a compound of formula (I)

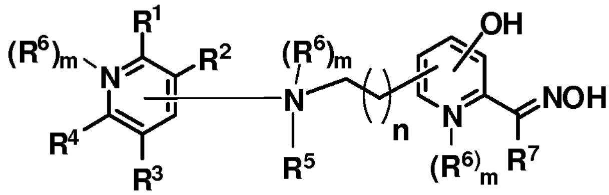

(I)

wherein the different groups are as defined in the detailed description below.
Another object of the present invention is a pharmaceutical composition comprising at least one compound of formula (I) and at least one pharmaceutically acceptable support. Another object of the invention is a compound according to the invention, for use as a medicine.
A further object of the invention is a compound according to the invention for use in the treatment of a nervous and/or respiratory failure due to intoxication with at least one organophosphorous nerve agent.

Still a further object of this invention is a compound of Formula (I) for use in the treatment of neurological diseases such as Alzheimer's disease.

## Detailed description of the invention

A first object of the invention is a compound of formula (I):

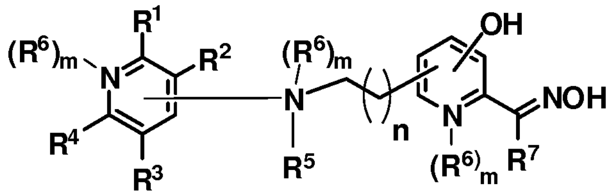

(I)

wherein:

n is 0, 1, 2, 3 or 4
m is 0 or 1,
$R^1$ and $R^2$ form together and with the carbon atoms linked thereto an aryl group, preferably a substituted or a non-substituted phenyl group, thereby forming a quinoline structure,
$R^3$ and $R^4$ are independently selected from the group consisting of a hydrogen atom, a halogen atom, an alkyl group, an alkoxy group, $CF_3$ and an aryl group,
$R^5$ is selected from the group consisting of a hydrogen atom, an alkyl group and an acyl group,
$R^6$ is a hydrogen atom or an alkyl group, when present, and
$R^7$ is selected from the group consisting of a hydrogen atom, a $NH_2$ group and a $CF_3$ group.

In the present invention, an "alkyl group" is a linear hydrocarbon group comprising from 1 to 6 carbon atoms, in particular from 1 to 3 carbon atoms, or a branched or cyclic hydrocarbon group comprising from 3 to 6 carbon atoms. Examples of alkyl groups include: methyl, ethyl, n-propyl, isopropyl, n-butyl, isobutyl, tert-butyl, n-pentyl, isopentyl, n-hexyl, and cyclohexyl groups, and preferably methyl.
An "alkoxy group" is an -O-alkyl group wherein the alkyl moiety is as defined above.
An "aryl group" refers to a monocyclic or polycyclic hydrocarbon aromatic group, with at least one of the rings having a

system of conjugated pi electrons, which may be optionally substituted. Examples of aryl groups that can be used are optionally substituted phenyl, naphthalene and anthracene groups. Preferably, the aryl group is a phenyl group, which may be unsubstituted or substituted with one or more substituents independently selected from a halogen atom, an alkyl group, an alkoxy group and a $CF_3$ group.
A "heteroaryl group" is an aryl group, wherein at least one carbon atom is replaced with a heteroatom such as N, O, P or S. Examples of heteroaryl groups include pyrrole, thiophene, furane, pyridine, pyrimidine, pyrazine, triazine, imidazole, thiazole, oxazole, and isoxazole groups.
An "acyl group" is an alkyl-CO- group wherein the alkyl moiety is as defined above.
A "halogen atom" is an atom of F, Cl, Br or I.

The compound of formula (I) may be labeled with one or more isotopes such as $^{15}N$, $^{2}H$ (D) and $^{3}H$ (T).

According to a preferred embodiment of this invention, m is 0. Neutral compounds are indeed preferred in this invention because they are able to cross the blood-brain barrier more easily in order to reactivate OPNA-inhibited AChE. According to another embodiment of this invention, m = 1 and $R^6$ = H. In this situation, preferred counter ions are $Cl^-$, $Br^-$, $I^-$, $SO_4^{2-}$ , $AcO^-$, $CF_3CO_2^-$, $BzO^-$, $CH_3SO_3^-$ and $CF_3SO_3^-$.
Moreover, it is preferred that at least one and preferably all the following conditions are met:

- n is 2, 3 or 4, preferably n is 3,
- $R^1$ and $R^2$ form together and with the carbon atoms linked thereto an aryl group, preferably a substituted or non-substituted phenyl group, thereby forming a quinoline structure,
- at least one, preferably at least two and more preferably each of $R^3$, $R^5$ and $R^7$ is a hydrogen atom, preferably a hydrogen atom,
- $R^4$ is a hydrogen atom or an alkyl group such as a methyl group,
- the pyridine ring in Formula (I) is substituted in position 6 by the quinoline-bearing group and/or in position 3 by the OH group.

Alternatively, the compound according to this invention may be such that at least one and preferably all the following conditions are met:

- n is 2, 3 or 4, preferably n is 2 or 3,
- at least one, preferably at least two and more preferably each of $R^3$, $R^5$ and $R^7$ is a hydrogen atom,
- $R^4$ is a hydrogen atom or an alkyl group such as a methyl group, preferably a hydrogen atom,
- the hydroxy-pyridine ring in Formula (I) is substituted in position 6 by the pyridine-bearing group and/or in position 3 by the OH group.

For instance, the compound according to this invention may have the following formula (Ia):

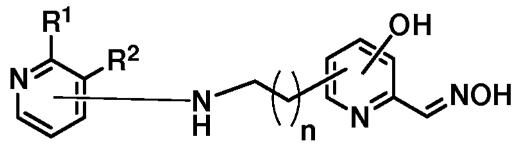

(Ia)

in which n is 2 or 3 and $R^1$ and $R^2$ form together and with the carbon atoms linked thereto a phenyl group which may be substituted or non substituted.

The compounds of the invention are preferably selected from those listed in Table 1 below.

Table 1

| Exemplary reactivators | | |
|---|---|---|
| A | 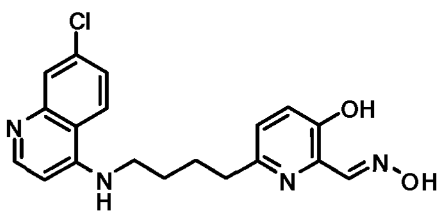 | (E)-6-(4-((7-chloroquinolin-4-yl) amino)butyl)-3-hydroxypicolinaldehyde oxime |

(continued)

| Exemplary reactivators | | |
|---|---|---|
| B | 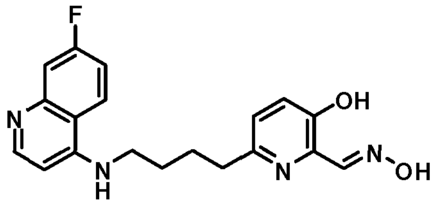 | (E)-6-(4-((7-fluoroquinolin-4-yl) amino)butyl)-3-hydroxypicolinaldehyde oxime |
| C | 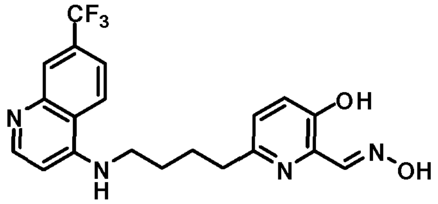 | (E)-6-(4-((7-(trifluoromethyl)quinolin-4-yl)amino)butyl)-3-hydroxy picolinaldehyde oxime |
| D | 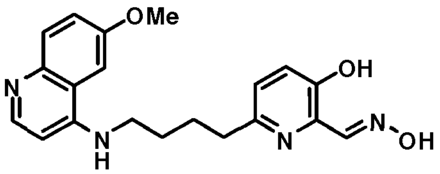 | (E)-6-(4-((6-methoxyquinolin-4-yl) amino)butyl)-3-hydroxypicolinaldehyde oxime |
| E | 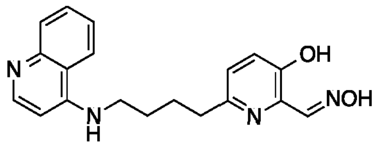 | (E)-6-(4-(quinolin-4-yl)amino)butyl)-3-hydroxy picolinaldehyde oxime |
| F | 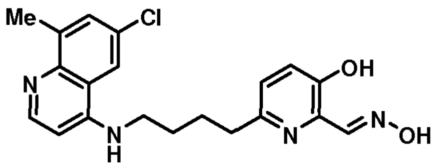 | (E)-6-(4-((6-chloro-8-methylquinolin-4-yl)amino)butyl)-3-hydroxy picolinaldehyde oxime |
| G | 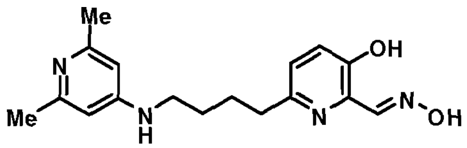 Reference compound | (E)-6-(4-((2,6-dimethylpyridin-4-yl) amino)butyl)-3-hydroxypicolinaldehyde oxime |
| H | 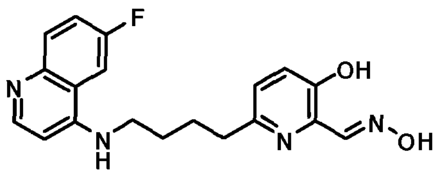 | (E)-6-(4-((6-fluoroquinolin-4-yl)amino)butyl)-3-hydroxy picolinaldehyde oxime |
| I | 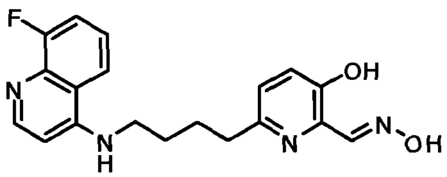 | (E)-6-(4-((8-fluoroquinolin-4-yl)amino)butyl)-3-hydroxy picolinaldehyde oxime |

(continued)

| Exemplary reactivators | | |
|---|---|---|
| J | 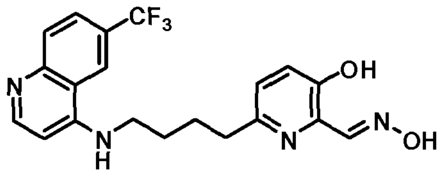 | (E)-6-(4-((6-(trifluoromethyl)quinolin-4-yl)amino)butyl)-3-hydroxy picolinaldehyde oxime |
| K | 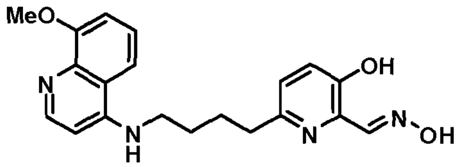 | (E)-6-(4-((8-methoxyquinolin-4-yl) amino)butyl)-3-hydroxypicolinaldehyde oxime |
| L | 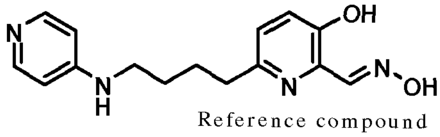 Reference compound | (E)-6-(4-(pyridin-4-ylamino)butyl)-3-hydroxypicolinaldehyde oxime |
| M | 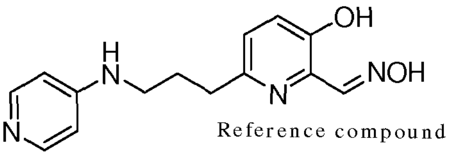 Reference compound | (E)-6-(4-(pyridin-4-ylamino)propyl)-3-hydroxypicolinaldehyde oxime |

In a specific embodiment, the compound of formula (I) is compound **E**

*Synthesis of the compounds of the invention*

A compound of formula (I) according to the invention may be synthesized by any appropriate method known by anyone of ordinary skill in the art.
In particular, the process for the synthesis of compounds of formula (I) may comprise a step of Sonogashira coupling reaction between a halogenated pyridine derivative or a halogenated quinoline moiety, and an amino-compound comprising a terminal alkyne. Alternatively, an aromatic nucleophilic substitution (SNAr) reaction may be used with the same reagents, i.e. between a halopyridine scaffold or a halogenated quinoline moiety, and an amino-compound bearing a terminal alkyne. In the particular case of aminopyridine derivatives, the alkylation of the amino- group with an electrophilic alkyl-halide compound comprising a terminal alkyne can be used. The resulting alkyne may be reduced by reaction with hydrogen, for instance in presence of Pearlman's catalyst $Pd(OH)_2/C$.
For the compounds of formula (I) with $R^7$ = H, the oxime may be formed from the corresponding aldehyde by reaction with $NH_2OH$, preferably as a hydrochloride, preferably in presence of a base, such as CH3COONa.
For the compounds of formula (I) with $R^7 = NH_2$, the amidoxime may be formed from the corresponding nitrile by reaction with $NH_2OH$, preferably as a hydrochloride, preferably in presence of a base, such as pyridine.

*Pharmaceutical use of the compounds of the invention*

The compounds of this invention may be used in the treatment of a nervous and/or respiratory failure due to intoxication with at least one organophosphorous nerve agent which may preferably be selected from warfare agents such as O-ethyl S-[2-(diisopropylamino)ethyl] methylphosphonothioate (VX), tabun, sarin, cyclosarin and soman and pesticides such as paraoxon, parathion and tetraethyl pyrophosphate (TEPP). These compounds may alternatively be used in the treatment of diseases, which involve a reduced production of acetylcholine that may be overcome by the administration of acetylcholinesterase inhibitors. Examples of such diseases include in particular neurological diseases such as Alzheimer's disease.
The compound of this invention is usually included in a pharmaceutical composition comprising at least one compound according to the invention and a pharmaceutically acceptable support.
The amount of compound of formula (I) in the composition according to the invention may vary in a broad range depending

upon the patient, the mode of administration and the expected effect.

The compound or composition according to the invention can be administered orally or non-orally, for instance *via* parenteral, intramuscular, intravenous, cutaneous, nasal or rectal route.

The pharmaceutical composition of the invention can present different forms including granules, powders, tablets, capsules, syrups, emulsions, suspensions, and forms used for non-oral administration, for instance injections, sprays, transdermal patches or suppositories. These pharmaceutical forms can be prepared *via* known conventional techniques.

The preparation of an orally administered solid pharmaceutical form can be for instance performed by the following process: an excipient (for example lactose, sucrose, starch or mannitol), a disintegrant (for example calcium carbonate, calcium carboxymethylcellulose, alginic acid, sodium carboxymethylcellulose, colloidal silicon dioxide, sodium croscarmellose, Crospovidone, guar gum, magnesium aluminium silicate, microcrystalline cellulose, cellulose powder, pregelatinised starch, sodium alginate or starch glycolate), a binder (for example alpha-starch, gum arabic, carboxymethylcellulose, polyvinylpyrrolidone, hydroxypropylcellulose, alginic acid, carbomer, dextrin, ethylcellulose, sodium alginate, maltodextrin, liquid glucose, magnesium aluminium silicate, hydroxyethylcellulose, methylcellulose or guar gum) and a lubricant (for example talc, magnesium stearate or polyethylene 6000) are added to the active principle and the mixture obtained is then tabletted. If necessary, the tablet can be coated via the known techniques, in order to mask the taste (for example with cocoa powder, mint, borneol or cinnamon powder) or to allow enteric dissolution or sustained release of the active principles. Coating products that can be used are, for example, ethylcellulose, hydroxymethylcellulose, polyoxyethylene glycol, cellulose acetophthalate, hydroxypropylmethylcellulose phthalate and Eudragit® (methacrylic acid-acrylic acid copolymer), Opadry® (hydroxypropylmethylcellulose + macrogol + titanium oxide + lactose monohydrate). Pharmaceutically acceptable colorants may be added (for example yellow iron oxide, red iron oxide or quinoline yellow lake).

Liquid pharmaceutical forms for oral administration include solutions, suspensions and emulsions. The aqueous solutions can be obtained by dissolving the active principle in water, followed by addition of flavourings, colorants, stabilisers and/or thickeners, if necessary. In order to improve the solubility, it is possible to add ethanol, propylene glycol or any other pharmaceutically acceptable non-aqueous solvent. The aqueous suspensions for oral use can be obtained by dispersing the finely divided active principle in water with a viscous product, such as a natural or synthetic gum or resin, methylcellulose or sodium carboxymethylcellulose.

The pharmaceutical forms for injection can be obtained, for example, by the following process. The active principle is dissolved, suspended or emulsified either in an aqueous medium (for example distilled water, physiological saline or Ringer's solution) or in an oily medium (for example olive oil, sesame seed oil, cottonseed oil, corn oil or propylene glycol), with a dispersant (for example Tween® 80, HCO® 60 (Nikko Chemicals), polyethylene glycol, carboxymethylcellulose or sodium alginate), a preserving agent (for example methyl p-hydroxybenzoate, propyl p-hydroxybenzoate, benzyl alcohol, chlorobutanol or phenol), an isotonicity agent (for example sodium chloride, glycerol, sorbitol or glucose) and optionally other additives, such as, if desired, a solubilising agent (for example sodium salicylate or sodium acetate) or a stabiliser (for example human serum albumin).

Pharmaceutical forms for external use can be obtained from a solid, semi-solid or liquid composition containing the active principle. For example, to obtain a solid form, the active principle can be treated with excipients (for example lactose, mannitol, starch, microcrystalline cellulose or sucrose) and a thickener (for example natural gums, cellulose derivatives or acrylic polymers) so as to convert them into powder. The liquid pharmaceutical compositions are prepared in substantially the same way as the forms for injection, as indicated previously. The semi-solid pharmaceutical forms are preferably in the form of aqueous or oily gels or in the form of pomades. These compositions may optionally contain a pH regulator (for example carbonic acid, phosphoric acid, citric acid, hydrochloric acid or sodium hydroxide) and a preserving agent (for example a p-hydroxybenzoic acid ester, chlorobutanol or benzalkonium chloride).

A method for the treatment of a nervous and/or respiratory failure due to intoxication with at least one organophosphorous nerve agent, comprising administering at least one compound according to the invention is also described herein, as well as the use of a compound according to the invention for the preparation of a medicine for the treatment of a nervous and/or respiratory failure due to intoxication with at least one organophosphorous nerve agent.

Within the context of the invention, the term treatment denotes curative, symptomatic, and/or preventive treatments. In particular, it can refer to reducing the progression of the disease, reducing or suppressing at least one of its symptoms or complications, or improving in any way the state of health of patients.

The administration of the compounds or of the composition according to the invention may be performed before, during or after the exposition of the subject to the organophosphorous nerve agent.

In the present invention, the terms "subject" and "patient" are used indifferently and designate a human subject.

The amount of compound of formula (I) to be administered according to the invention may vary in a broad range depending upon the patient, the mode of administration and the expected effect. In particular, the amount of compound of formula (I) may be comprised between 200 mg and 4000 mg, with up to 3 daily intakes.

The compound or composition according to the invention may be co-administered with at least one other active agent, such as an antimuscarinic agent, in particular atropine, an anticonvulsant, in particular diazepam or one of its

prodrugs, such as avizafone, and/or a bioscavenger able to capture and/or degrade OPNAs in blood, such as human butyrylcholinesterase.

The term co-administered means that the administration of the compound or composition according to the invention and that of the other active agent can be simultaneous, sequential and /or separate.

*Other uses of the compounds of the invention*

The compounds of this invention may further be used as screening tools for the identification of more potent hAChE reactivators. In this application, the compounds of formula (I) may include one or more isotopes, which will allow for their detection.

The following examples are provided as illustrative, and not limitative, of the present invention.

**Examples:**

Example 1 : Synthesis of Compound **E** according to the invention

General procedure

Starting from the commercially available 4-bromoquinoline **1,** a Buchwald-Hartwig amination coupling was carried out with 1-amino-3-butyne to afford the terminal alkyne **2.** After filtration on silica, the crude was subjected to Sonogashira coupling to afford the intermediate alkyne **3** in good yield after 2 steps (60%). Then the aldehyde **4** was isolated in excellent yield (69%) using a three-step sequence including: (a) *"one-pot"* alkyne reduction and *O*-debenzylation; (b) TBS protection of the phenolic function using TBSOTf and 2,6-lutidine; (c) reduction of the methyl ester into the corresponding aldehyde using DIBAL-H and deprotection of the silyloxy function. Finally, the desired oxime **5** was isolated by oximation using hydroxylamine hydrochloride and anhydrous sodium acetate in ethanol. It was recovered in 54% yield after purification by column chromatography and crystallization.

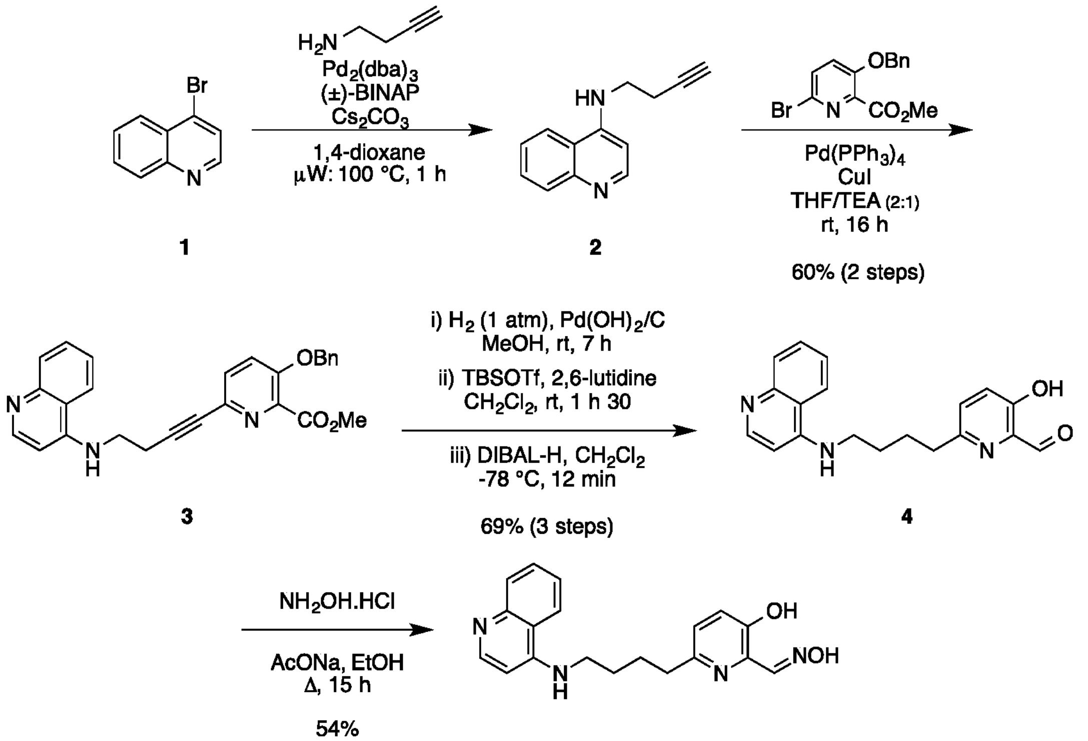


**Scheme 1 Synthesis of hybrid reactivator E.**

**Methyl 3-(benzyloxy)-6-(4-(quinolin-4-ylamino)but-1-yn-1-yl)picolinate (3).**

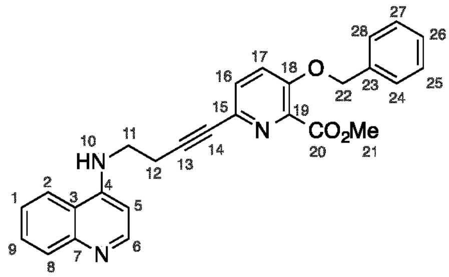

$C_{27}H_{23}N_3O_3$
MW: 437.50 $g.mol^{-1}$

A microwave tube containing a magnetic stirrer bar was charged with 4-bromoquinoline (143 mg, 0.690 mmol, 1.0 equiv), $Pd_2(dba)_3$ (25 mg, 0.028 mmol, 0.04 equiv), (±)-BINAP (37 mg, 0.055 mmol, 0.08 equiv) and $C_{S2}CO_3$ (562 mg, 1.725 mmol, 2.5 equiv). The vessel was sealed with a microwave septum and purged with argon. Degassed 1,4-dioxane (2.3 mL) and 1-amino-3-butyne (73 μL, 0.897 mmol, 1.3 equiv) were introduced through the septum. The resulting mixture was heated to 100 °C for 1 h using a Biotage® Initiator Microwave Synthesizer Apparatus. After cooling, the reaction mixture was concentrated and filtrated through a small plug of silica. The crude material was directly used to the subsequent step.

To a degassed solution of methyl 3-benzyloxy-6-bromopicolinate (244 mg, 0.759 mmol, 1.1 equiv) in THF/$Et_3N$ (7 mL/ 5 mL), $Pd[PPh_3]_4$ (79 mg, 0.069 mmol, 0.1 equiv) and CuI (26 mg, 0.138 mmol, 0.2 equiv) were added. After degasing the reaction mixture for 5 min at room temperature, a degassed solution of the previous *N*-(but-3-yn-1-yl)quinolin-4-amine 2 (0.690 mmol, 1 equiv) in THF (3 mL) was added dropwise and the reaction mixture was stirred at room temperature for 16 h. After completion, the reaction mixture was concentrated under reduced pressure and the residue was purified by column chromatography ($CH_2Cl_2$/MeOH 9:1) to afford the desired methyl 3-(benzyloxy)-6-(4-(quinolin-4-ylamino)but-1-yn-1-yl)picolinate **3** as white foam (181 mg, 0.414 mmol, 60%).

**TLC $R_f$** ($CH_2Cl_2$/MeOH 8:2) 0.30.

**IR $v_{max}$ neat ($cm^{-1}$)** 3234 (m, b), 3034 (b), 2950 (m), 2881 (m), 2231 (w), 1733 (s), 1583 (s), 1451 (s), 1383 (m), 1341 (m), 1273 (s), 1211 (s), 1099 (s).

**$^1$H NMR (400 MHz, $CDCl_3$) δ (ppm)** 8.38 (d, *J* = 6.1 Hz, 1H, **6**), 8.33 (d, *J* = 8.3 Hz, 1H, **8**), 8.07 (d, *J* = 8.6 Hz, 1H, **2**), 7.66 (br s, 1H, **10**), 7.64 (t, *J* = 7.7 Hz, 1H, **9**), 7.49-7.26 (m, 8H, **1+16+17+24+25+26+27+28**), 6.54 (d, *J* = 6.4 Hz, 1H, **5**), 5.18 (s, 2H, **22**), 3.94 (s, 3H, **21**), 3.72 (q, *J* = 6.0 Hz, 2H, **11**), 2.89 (t, *J* = 6.9 Hz, 2H, **12**).

**$^{13}$C NMR (101 MHz, $CDCl_3$) δ (ppm)** 164.98 (**20**), 153.46 (**6**), 153.12 (**18**), 145.67 (7), 142.91 **(4),** 140.26 **(19),** 135.50 **(15),** 134.66 **(23),** 131.52 **(9),** 130.27 **(16),** 128.89 (2C, **25+27**), 128.45 **(26),** 127.06 (2C, **24+28),** 126.14 **(8),** 124.93 **(1),** 121.99 **(17),** 121.95 **(2),** 118.13 **(3),** 98.26 **(5),** 86.31 **(16),** 81.50 **(14),** 71.01 **(22),** 52.90 **(21),** 41.93 **(11),** 19.76 **(12).**

**LRMS (ESI$^+$)** *m/z* 438.0 ($[M+H]^+$).
**HRMS (ESI$^+$)** *m/z* calcd for $C_{27}H_{24}N_3O_3^+$ 438.1812 found 438.1804 Da.

**3-Hydroxy-6-(4-(quinolin-4-ylamino)butyl)picolinaldehyde (4).**

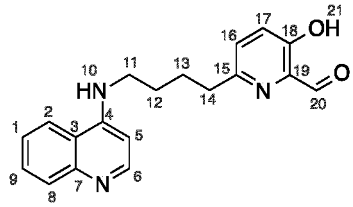

$C_{19}H_{19}N_3O_2$
MW: 321.38 $g.mol^{-1}$

To a degassed solution of methyl 3-(benzyloxy)-6-(4-(quinolin-4-ylamino)but-1-yn-1-yl)picolinate **3** (358 mg, 0.820 mmol, 1.0 equiv) in dry MeOH (60 mL), Pearlman's catalyst $Pd(OH)_2/C$ (20% wt. with 50% moisture, 230 mg,

0.164 mmol, 0.2 equiv) was added. After evaporating and flushing with $H_2$ five times, the reaction mixture was stirred for 7 h at room temperature under $H_2$ (1 atm.). Upon completion, the catalyst was removed by filtration through a short column of celite, the solvent was evaporated and the residue was directly subjected to the following step without purification. The presence of the desired reduced product was supported by TLC and LRMS (ESI$^+$) *m/z* 351.9 ([M+H]$^+$).

To a solution of methyl 3-hydroxy-6-(4-(quinolin-4-ylamino)butyl)picolinate (288 mg, 0.820 mmol, 1.0 equiv) in dry $CH_2Cl_2$ (8.2 mL), 2,6-lutidine (286 μL, 2.459 mmol, 3.0 equiv), and *tert*-butyldimethylsilyltrifluoromethanesulfonate (564 μL, 2.459 mmol, 3.0 equiv) were successively added and the reaction mixture was stirred at room temperature under argon atmosphere for 1 h 30. After completion, the reaction mixture was quenched with a saturated aqueous $CuSO_4$ solution (10 mL). After separation, the aqueous phase was extracted with $CH_2Cl_2$ and the combined organic was then dried over $Na_2SO_4$ and concentrated under reduced pressure. The presence of the desired silylether was supported by TLC and LRMS (ESI$^+$) *m/z* 466.1 ([M+H]$^+$). After drying *in vacuo,* the residue was directly subjected to the following step.

To the solution of methyl 3-((*tert*-butyldimethylsilyl)oxy)-6-(4-(quinolin-4-ylamino)butyl)picolinate (0.820 mmol, 1.0 equiv) in dry $CH_2Cl_2$ (8.2 mL) at -78 °C, DIBAL-H (2.46 mL of a 1.0 M solution in $CH_2Cl_2$, 2.459 mmol, 3.0 equiv) was added dropwise. The reaction mixture was stirred at -78 °C for 12 min, then the reaction was quenched with MeOH (3 mL), and the cooling bath was removed. When the mixture warmed to room temperature the organic phase was washed with an aqueous solution of NaOH (1.0 M). The aqueous phase was then extracted with $CH_2Cl_2$ and the organics were dried over $Na_2SO_4$ and concentrated under reduced pressure. After drying *in vacuo,* the residue was purified by column chromatography ($CH_2Cl_2$/MeOH 8:2) to afford the desired 3-hydroxy-6-(4-(quinolin-4-ylamino)butyl)picolinaldehyde **4** as bright yellow wax (182 mg, 0.566 mmol, 69% over 3 steps).

**TLC *R*$_f$** ($CH_2Cl_2$/MeOH 8:2) 0.14.

**IR $v_{max}$ neat (cm$^{-1}$)** 3275 (b), 2924 (m), 2853 (m), 1668 (m), 1584 (s), 1457 (s), 1340 (m), 1223 (m), 1139 (m).

**$^1$H NMR (400 MHz, $CDCl_3$)** δ **(ppm)** 10.66 (br s, 1H, **21**), 10.04 (s, 1H, **20**), 8.56 (d, *J* = 4.9 Hz, 1H, **6**), 7.98 (d, *J* = 8.6 Hz, 1H, **8**), 7.74 (d, *J* = 8.1 Hz, 1H, **2**), 7.63 (t, *J* = 7.6 Hz, 1H, **9**), 7.42 (t, *J* = 7.5 Hz, 1H, **1**), 7.34-7.29 (m, 2H, **16+17**), 6.43 (d, *J* = 5.1 Hz, 1H, **5**), 5.07 (br s, 1H, **10**), 3.39 (q, *J* = 6.6 Hz, 2H, **11**), 2.90 (t, *J* = 7.5 Hz, 2H, **14**), 2.05-1.79 (m, 4H, **12+13**).

**$^{13}$C NMR (101 MHz, $CDCl_3$)** δ **(ppm)** 198.75 **(20),** 154.27 **(18),** 151.27 **(6),** 149.69 **(4),** 130.31 **(9),** 129.95 **(8),** 129.14 **(1),** 126.76 **(16),** 124.76 **(2),** 119.28 **(17),** 98.98 **(5),** 43.31 **(11),** 36.88 **(14),** 28.40 **(12),** 27.28 **(13).** Short run was made due to the stability of aldehyde, thus some quaternary carbons were not visible **(3, 7, 15** and **19)**

**LRMS (ESI$^+$)** *m/z* 322.0 ([M+H]$^+$).
**HRMS (ESI$^+$)** *m/z* calcd for $C_{19}H_{20}N_3O_2^+$ 322.1550 found 322.1544 Da

**3-Hydroxy-6-(4-(quinolin-4-ylamino)butyl)picolinaldehyde oxime acetate (E).**

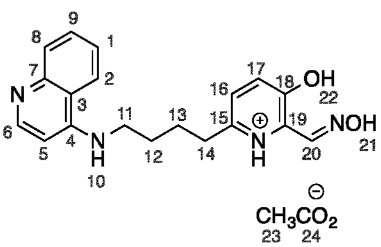

$C_{19}H_{20}N_4O_2$ . $C_2H_4O_2$
MW: 336.40 g.mol$^{-1}$
MW acetate: 396.45 g.mol$^{-1}$

A solution of 3-hydroxy-6-(4-(quinolin-4-ylamino)butyl)picolinaldehyde 4 (102 mg, 0.319 mmol, 1.0 equiv), hydroxylamine hydrochloride (33 mg, 0.479 mmol, 1.5 equiv), and $CH_3CO_2Na$ (39 mg, 0.479 mmol, 1.5 equiv) in dry EtOH (10 mL) was stirred at reflux for 15 h. After concentration under reduced pressure, the crude product was purified by the column chromatography ($CH_2Cl_2$/MeOH 9:1) and precipitated in methanol/hexane (2:3) to afford the desired 3-hydroxy-6-(4-(quinolin-4-ylamino)butyl)picolinaldehyde oxime as white solid (58 mg, 0.037 mmol, 54%).

**TLC *R*$_f$** ($CH_2Cl_2$/MeOH 8:2) 0.13.
**MP** 197 °C (methanol/hexane 2:3)

**IR $v_{max}$ neat ($cm^{-1}$)** 3420 (w), 2928 (w), 2860 (w), 2570 (w, b), 1586 (s), 1538 (s), 1460 (m), 1347 (m), 1262 (s), 1037 (s).
**$^1$H NMR (500 MHz, $CD_3OD$) δ (ppm)** 8.33 (d, *J* = 6.5 Hz, 1H, **6**), 8.25 (dt, *J* = 8.5, 1.0 Hz, 1H, **8**), 8.23 (s, 1H, **20**), 7.83-7.79 (m, 2H, **2+9**), 7.63-7.56 (m, 1H, **1**), 7.25 (d, *J* = 8.5 Hz, 1H, **17**), 7.16 (d, *J* = 8.5 Hz, 1H, **16**), 6.69 (d, *J* = 6.6 Hz, 1H, **5**), 3.54 (t, *J* = 6.8 Hz, 2H, **11**), 2.81 (t, *J* = 7.2 Hz, 2H, **14**), 1.92 (s, 3H, **23**), 1.90-1.77 (m, 4H, **12+13**).
**$^{13}$C NMR (125.8 MHz, $CD_3OD$) δ (ppm)** 155.80 **(4),** 154.21 **(15),** 153.85 **(18),** 152.71 **(20),** 146.03 **(6),** 142.88 **(7),** 136.38 **(19),** 133.21 **(9),** 127.18 **(1),** 126.05 **(17),** 125.45 **(16),** 123.98 **(2),** 123.14 **(8),** 119.09 **(3),** 99.17 **(5),** 44.18 **(11),** 37.27 **(14),** 28.47 **(12),** 28.41 **(13),** 23.38 **(23).**

**LRMS (ESI$^+$)** *m*/*z* 337.1 ([M+H]$^+$).
**HRMS (ESI$^+$)** *m*/*z* calcd for $C_{19}H_{21}N_4O_2^+$ 337.1659 found 337.1657 Da

Synthesis of the bis acetate of Compound **E**

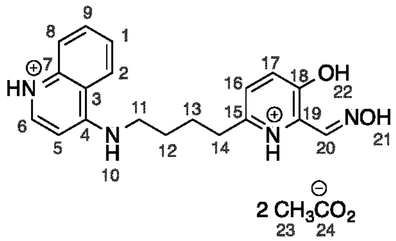

Compound **E** was dissolved in glacial acetic acid and stirred for 10 min at room temperature. Lyophilisation of the homogenous solution led to the acetate salt of **E.** Alternatively, the solution can be concentrated using a rotavapor and the residue can be dried in vacuum to afford the desired compound.

**$^1$H NMR** **(500 MHz, $CD_3OD$) δ (ppm)** 8.33 (d, *J* = 6.6 Hz, 1H, **6**), 8.29 (m, 1H, **8**), 8.21 (s, 1H, **20**), 7.89-7.85 (m, 1H, **9**), 7.83-7.81 (m, 1H, **2**), 7.64 (ddd, *J* = 8.4, 6.9, 1.4 Hz, 1H, **1**), 7.25 (d, *J* = 8.3 Hz, 1H, **17**), 7.16 (d, *J* = 8.3 Hz, 1H, **16**), 6.74 (d, *J* = 6.7 Hz, 1H, **5**), 3.57 (t, *J* = 6.8 Hz, 2H, **11**), 2.81 (t, *J* = 7.2 Hz, 2H, **14**), 1.93 (s, 6H, **23**), 1.90-1.81 (m, 4H, **12+13).**

**$^{13}$C NMR** **(125.8 MHz, $CD_3OD$) δ (ppm)** 156.56 **(4),** 154.15 **(15),** 153.84 **(18),** 152.68 **(20),** 144.77 **(6),** 136.39 **(19),** 133.88 **(9),** 127.58 **(1),** 126.05 **(17),** 125.46 **(16),** 123.37 **(2),** 122.80 **(8),** 118.80 **(3),** 99.17 **(5),** 44.30 **(11),** 37.23 **(14),** 28.39 **(12),** 28.34 **(13),** 23.02 **(23).**

Example 2: Synthesis of Compound M according to the invention

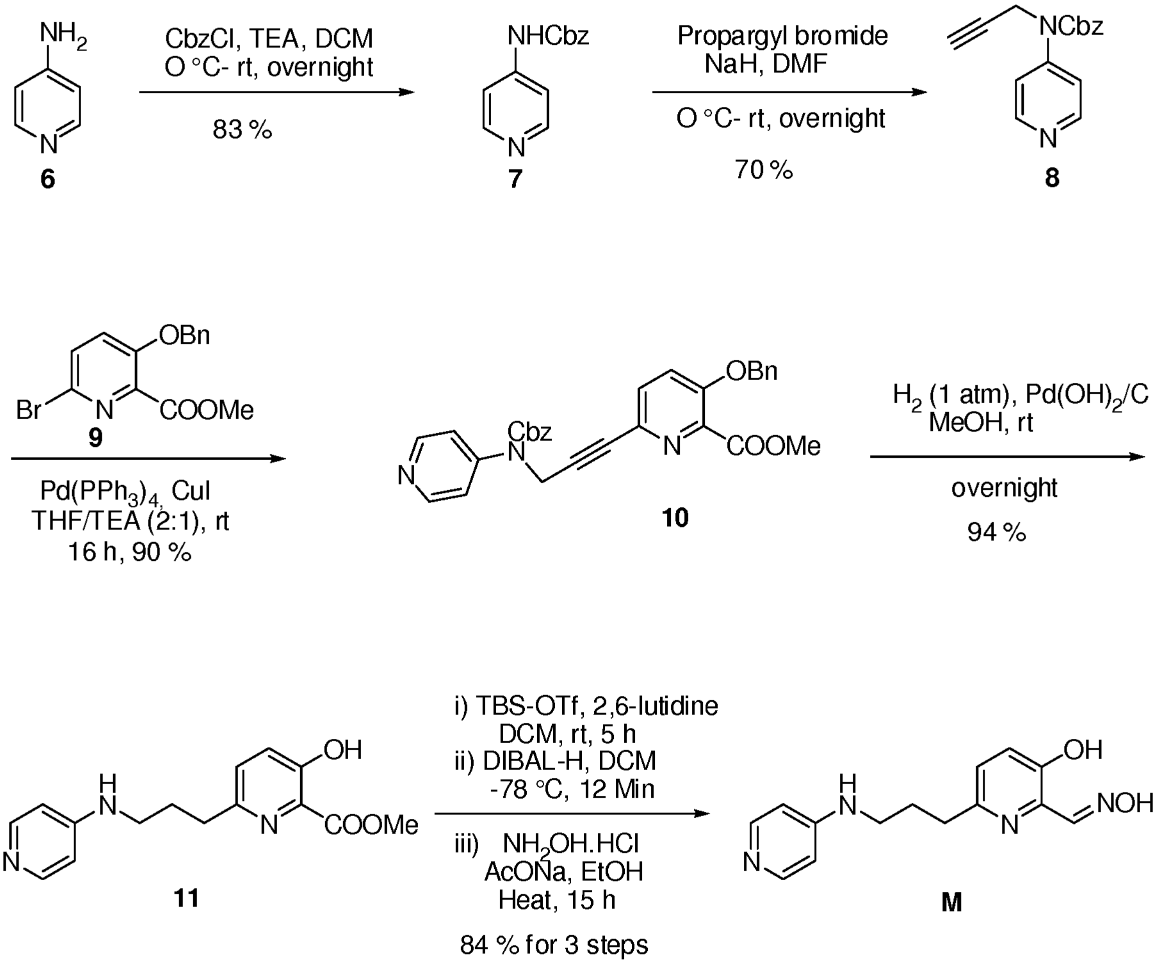


Scheme 2: Synthesis of hybrid reactivator **M**

**Synthesis of N-(4-Pyridyl)Benzyl Carbamate (7):**

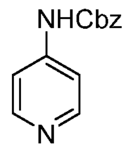

To a solution of 4-aminopyridine 1 (3.2 g, 34.0 mmol) in $CH_2Cl_2$ (80 mL) at 0° C. was added triethylamine (9.50 mL, 68.0 mmol) and benzyl chloroformate (7.3 mL, 51.0 mmol). The ice bath was removed and the resulting mixture was allowed to warm to roomtemperature overnight before being poured into saturated aqueous $NaHCO_3$. The product was extracted with $CH_2Cl_2$, dried ($Na_2SO_4$) and concentrated. The crude product was recrystallized from EtOAc to give 6.4 g (83% yield) of pure benzyl carbamate 7. $^1H$ NMR (400 MHz, $CDCl_3$) $\delta$ (ppm) 5.22 (s, 2H), 7.32-7.42 (m, 7H), 8.45 (d, *J* = 5.9 Hz, 2H).
*Ref* From U.S. Pat. Appl. Publ., 20110130429, 02 Jun 2011

**Synthesis of benzyl prop-2-yn-1-yl(pyridin-4-yl)carbamate (8):**

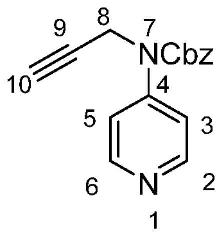

To a stirred solution of NaH (632 mg, 26.30 mmol) in DMF (15 mL) at 0° C. was added benzyl carbamate 7 (2 g, 8.77 mmol) in DMF (15 mL) and stirred it for 30 min at 0° C. Then propargyl bromide (1.15 mL, 13.15 mmol) was added slowly at 0° C. Warmed the reaction mixture to room temperature and stirred it for 4 h. after completion of the reaction, the reaction mixture was quenched with saturated $NH_4Cl$ solution and extracted with EtOAc. The combined organic layers are dried over $MgSO_4$. The solids were filtered off and the solvent is evaporated. The crude product was purified by silica gel column chromatography (EtOAc/cyclo hexane 1:4) to give the propargyl carbamate **8** (1.64 g, 70 %) as a white solid.

***R*** $_f$ (pure EtOAc) 0.60; **IR** (neat) $\nu_{max}$ 3165, 1717, 1589, 1389, 1212, 1040, 751, 695, 548, 506 $cm^{-1}$; **$^1$H NMR** (400 MHz, $CDCl_3$) $\delta$ (ppm) 8.54 (d, *J* = 5.5 Hz, 2H, Ar), 7.42-7.29 (m, 7H, Ar), 5.25 (s, 2H, $-CH_2Ph$), 4.49 (d, *J* = 2.4 Hz, 2H, $H_8$), 2.34 (t, *J* = 2.4 Hz, 1H, $H_{10}$). **$^{13}$C NMR** (100 MHz, $CDCl_3$) $\delta$ (ppm) 153.57, 150. 36, 148.56, 135.39, 128.49, 128.29, 127.95, 118.17 (Ar), 78.58 (C9), 73.03 (C10), 68.31 ($\underline{C}H_2Ph$), 38.69 (C8); **HRMS (ESI$^+$)** *m/z* calcd for $C_{16}H_{15}N_2O_2^+$ 267.1103 found 267.1128.

**Synthesis of methyl 3-(benzyloxy)-6-(3-(((benzyloxy)carbonyl)(pyridin-4-yl)amino)prop-1-yn-1-yl)picolinate (10):**

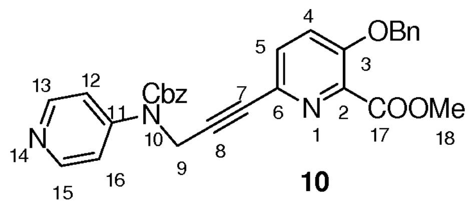

**10**

To a degassed solution of methyl 3-benzyloxy-6-bromopicolinate **9** (1.21 mg, 3.76 mmol, 1 equiv) in THF/$Et_3N$ (20 mL/ 20 mL), $Pd[PPh_3]_4$ (435 mg, 0.376 mmol, 0.1 equiv) and CuI (143 mg, 0.752 mmol, 0.2 equiv) were added. After degassing the reaction mixture for 5 min at room temperature, a degassed solution of the previous carbamate **8** (1 g, 3.76 mmol, 1 equiv) in THF (10 mL) was added dropwise and the reaction mixture was stirred at the room temperature for 16 h. After completion, the reaction mixture was concentrated under reduced pressure and the residue was purified by column chromatography ($CH_2Cl_2$/MeOH 9:1) to afford the desired picolinate **10** as white foam (1.71 g, 90%).

***R*** $_f$ (pure EtOAc) 0.36; **IR** (neat) $\nu_{max}$ 1722, 1588, 1387, 1209, 1097, 825, 734, 695 $cm^{-1}$; **$^1$H NMR** (400 MHz, $CDCl_3$) $\delta$ (ppm) 8.57 (d, *J* = 5.3 Hz, 2H, Ar), 7.50-7.23 (m, 15H, Ar), 5.32 (s, 2H, $-CH_2Ph$), 5.23 (s, 2H, $-COC\underline{H}_2Ph$), 4.74 (s, 2H, $H_9$), 3.99 (s, 3H, $H_{18}$); **$^{13}$C NMR** (100 MHz, $CDCl_3$) $\delta$ (ppm) 164.45 (C18), 153.43 ($-\underline{C}OCH_2Ph$), 153.25, 150.09, 148.17, 140.23, 135.35, 135.11, 133.60, 130.25, 128.56, 128.41, 128.17, 128.11, 127.92, 126.70, 121.39 (Ar), 83.41 (C7), 82.86 (C8), 70.61 ($-\underline{CH_2}Ph$), 68.22 ($-CO\underline{CH_2}Ph$), 52.55 (C18), 39.24 (C9); **HRMS (ESI$^+$)** *m/z* calcd for $C_{30}H_{26}N_3O_5^+$ 508.1844 found 508.1867.

**Synthesis of methyl 3-hydroxy-6-(3-(pyridin-4-ylamino)propyl)picolinate (11):**

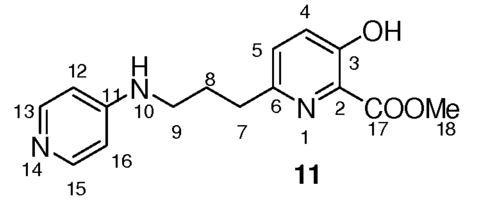

**11**

To a degassed solution of picolinate **10** (1.7 g, 0.820 mmol, 1 equiv) in dry MeOH (150 mL), Pearlman's catalyst $Pd(OH)_2/C$ (20% with 50% moisture, 188 mg, 1.342 mmol, 0.4 equiv) was added. After evaporating and flushing with $H_2$ three times, the reaction mixture was stirred at room temperature under $H_2$ (1 atm.) for 16 h. Upon completion, the catalyst was removed by filtration through a short column of celite, the solvent was evaporated, and the residue was directly subjected for the following step without purification.
***R*$_f$** (pure MeOH) 0.20; **IR** (neat) $v_{max}$ 2922, 2853, 1725, 1651, 1543, 1464, 1194, 1095, 792, 721, 673, 541, 507 $cm^{-1}$; **$^1$H NMR** (400 MHz, $CDCl_3$) $\delta$ (ppm) 8.90 (br t, *J* = 5.0 Hz, 1H, $H_{10}$), 7.90 (d, *J* = 6.3 Hz, 2H, $H_{13}$, $H_{15}$), 7.40 (d, *J* = 8.6 Hz, 1H, $H_8$), 7.30 (d, *J* = 8.6 Hz, 1H, $H_4$), 6.88, (br d, *J* = 5.0 Hz, 2H, $H_{12}$, $H_{16}$), 4.03 (s, 3H, $H_{18}$), 3.33 (q, *J* = 6.5 Hz, 2H, $H_9$), 2.95 (t, *J* = 7.1 Hz, 2H, $H_7$), 2.15 (p, *J* = 6.5 Hz, 7.0 Hz, 2H, $H_8$); **$^{13}$C NMR** (100 MHz, $CDCl_3$) $\delta$ (ppm) 169.61 (C17), 158.00, 157.32, 152.31, 139.09, 129.80, 127.11 (Ar), 53.17 (C18), 42.71 (C9), 34.30 (C7), 27.40 (C8); **HRMS (ESI$^+$)** *m/z* calcd for $C_{15}H_{18}N_3O_3^+$ 288.1330 found 288.1343.

**Synthesis of (*Z*)-3-hydroxy-6-(3-(pyridin-4-ylamino)propyl)picolinaldehyde oxime (M):**

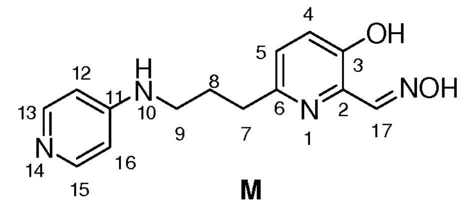

**M**

To a solution of deprotected picolinate (240 mg, 0.836 mmol, 1 equiv) in dry $CH_2Cl_2$ (5 mL), 2,6-lutidine (290 $\mu$L, 2.508 mmol, 3 equiv), and *tert*-butyldimethylsilyltrifluoromethanesul-fonate (293 $\mu$L, 1.672 mmol, 2 equiv) were successively added and the reaction mixture was stirred at the room temperature under argon atmosphere during 5 h. After completion, the reaction mixture was directly concentrated under reduced pressure to give silylated compound. LRMS (ESI$^+$) *m/z* 466.1 $[M+H]^+$. After drying *in vacuo,* the residue was directly subjected to the following step.

To the solution of silylated compound (160 mg 0.399 mmol, 1 equiv) in dry $CH_2Cl_2$ (5 mL) at -78 °C, DIBAL-H (1M solution in $CH_2Cl_2$, 2.99 mL, 2.394 mmol, 6 equiv) was added dropwise. The reaction mixture was stirred at -78 °C for 1 h, then the reaction was quenched with MeOH (3 mL), and the cooling bath was removed. When the mixture was warmed to room temperature the sovents in reaction mixture was evoporated under reduced pressure to give aldehyde compound as crude along with Aluminium salts. the salts were filtered by washing with $CH_2Cl_2$ (50 mL). The filtrate was evaporated, and the residue was directly subjected for the next step without purification.

A solution of picolinaldehyde (0.399 mmol, 1 equiv), hydroxylamine hydrochloride (56 mg, 0.798 mmol, 2 equiv), and $CH_3CO_2Na$ (65 mg, 0.798 mmol, 2 equiv) in dry ethanol (10 mL) was stirred at reflux during 16 h. After concentration under reduced pressure, the crude product was washed with $CH_2Cl_2$ (5 * 10 mL) to remove all the impurities carrying from the last two reactions. The existing compound in the round bottom flask was picolinaldehyde oxime, which was dried *in high vacuo* (95 mg, 84%) and confirmed by 1H NMR.
***R*$_f$** (pure MeOH) 0.20; **IR** (neat) $v_{max}$ 3282, 2944, 1650, 1544, 1257, 1228, 1170, 1023, 629, 515 $cm^{-1}$; **$^1$H NMR** (400 MHz, $CDCl_3$) $\delta$ (ppm) 8.21 (s, 1H, $H_{17}$), 7.97, 7.79 (2 br d, *J* = 6.6 Hz, 2H, $H_{13}$, $H_{15}$), 7.35, 7.27 (2 d, *J* = 8.6 Hz, 2H, $H_4$, $H_8$), 6.68, 6.57 (2 br d, *J* = 6.1 Hz, 2H, $H_{12}$, $H_{16}$), 3.36 (t, *J* = 6.5 Hz, 2H, $H_9$), 2.86 (t, *J* = 6.9 Hz, 2H, $H_7$), 2.08 (p, *J* = 6.5 Hz, 6.9 Hz, 2H, $H_8$); **$^{13}$C NMR** (100 MHz, $CDCl_3$) $\delta$ (ppm) 159.94 (-$\underline{C}OCH_3$), 152.74 (C17), 153.93, 153.41, 140.47, 136.63, 126.18, 125.63, 123.41, 120.25 (Ar), 43.35 (C9), 34.93 (C7), 29.48 (C8), 22.94 (-$CO\underline{C}H_3$); **HRMS (ESI+)** *m/z* calcd for $C_{14}H_{17}N_4O_2^+$ 273.1345 found 273.1346.

Example 3: Synthesis of Compound **K** according to the invention

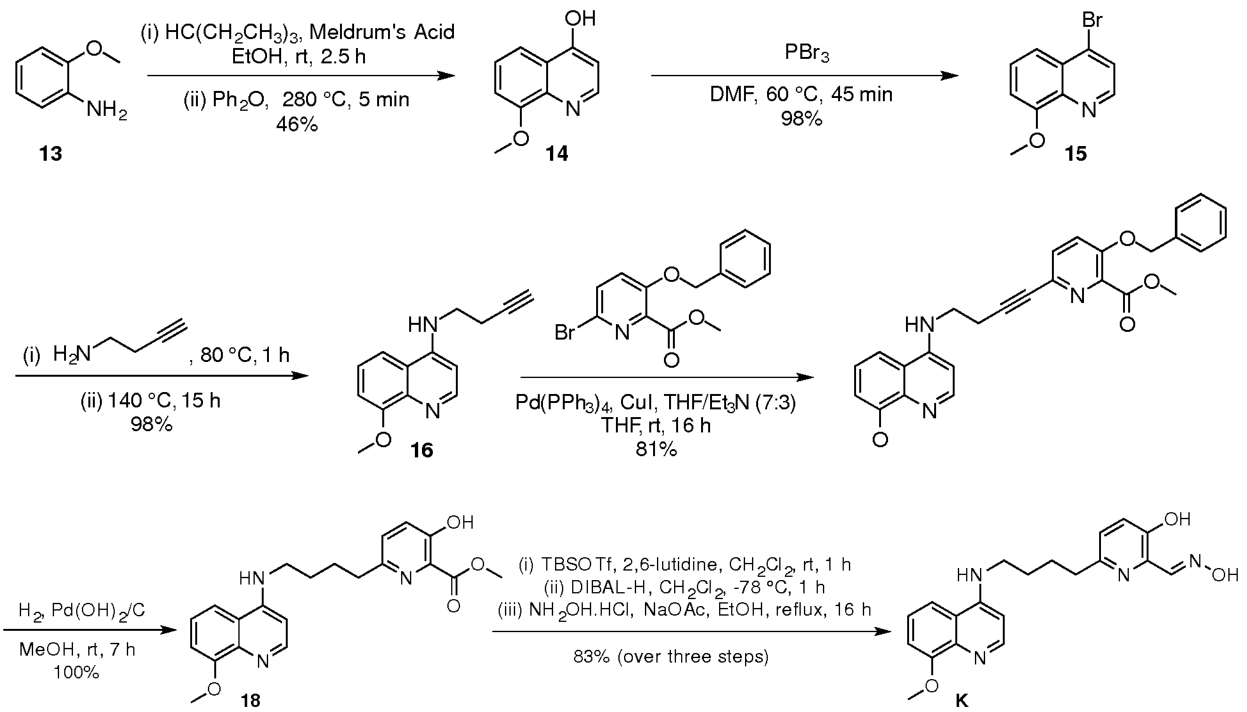


13 14 15 16 18 K

Scheme 2: Synthesis of hybrid reactivator **K**

**Synthesis of 8-methoxyquinolin-4-ol (14):**

To a solution of o-anisidine (2.29 mL, 20.3 mmol, 1 equiv) in ethanol (20 mL) were added Meldrum's acid (3.57 g, 24.8 mmol, 1.2 equiv) and triethyl orthoformate (8.0 mL, 48.0 mmol, 2.4 equiv). The mixture was heated to reflux for 2.5 h. The reaction was cooled to 0 °C and the solid was filtered and washed with cold ethanol. The dried solid was added portiowise over 5 mins to boiling diphenyl ether (100 mL). Reflux was maintained for an additional 3 mins. The reaction was stirred at room temperature for 30 mins before petroleum ether (25 mL) was added and the solid was filtered and dried. The crude product was purified by column chromatography on silica gel (EtOAc/MeOH 95:5), to afford 8-methoxyquinolin-4-ol (2.70 g, 15.4 mmol, 76%).

The analyses were consistent with the data reported in the literature.
*Ref:* WO2010/123995 (2010*)*

| | |
|---|---|
| **TLC $R_f$** | 0.06 (EtOAc/MeOH 95:5) |
| **$^1$HNMR** | (400MHz, DMSO-$d_6$) δ (ppm) 11.35 (s, 1H, **OH**), 7.58-7.70 (m, 1H, **NCH**), 7.38-7.43 (m, 1H, Ar**H**), 7.23-7.27 (m, 2H, OHCC**CH**, OMeC**CH**), 6.95-7.08 (m, 1H, OHC**CH**), 3.99 (s, 3H, **OMe**) ppm |
| **$^{13}$CNMR** | (101 MHz, DMSO-$d_6$) δ (ppm) 177.11 **(7),** 149.0 **(4),** 139.3 **(10),** 130.5 **(2),** 123.2 **(5),** 119.1 **(3),** 116.7 **(1),** 111.4 **(6),** 109.6 **(8),** 56.6 **(13)** ppm |
| **IR (neat)** | $v_{max}$ 2921 (br), 2851 (m), 1272 (s), 1041 (s) $cm^{-1}$ |
| **LCMS** | (ESI$^+$) *m/z* 176.1 $[M+H]^+$ (ESI$^+$) *m/z* 198.1 $[M+Na]^+$ |
| **HRMS** | (ESI$^+$) *m/z calcd.* 176.0706 *m/z meas.* 176.0707 $[M+H]^+$ |
| **Mpt** | 168 °C |

**Synthesis of 4-bromo-8-methoxyquinoline (15):**

To a solution of 8-methoxyquinolin-4-ol (2.67 g, 15.2 mmol, 1 equiv) in N,N-dimethylformamide (20 mL) at 60 °C was added phosphorus tribromide (1.65 mL, 17.5 mmol, 1.2 equiv). The mixture was cooled to 45 °C for 45 mins. After cooling to room temperature, the reaction was diluted with water (25 mL) and the pH was adjusted to ~ 10 with saturated aqueous $NaHCO_3$. The resulting solid was washed in water, filtered and taken up in EtOAc and concentrated *in vacuo,* to afford 8-methoxy-4-bromoquinoline (2.44 g, 10.3 mmol, 67%).

The analyses were consistent with the data reported in the literature.

*Ref:* WO2010/152603 (2008*)*

| | |
|---|---|
| **TLC $R_f$** | 0.51 (EtOAc/ MeOH 75:25) |
| **$^1$HNMR** | (400MHz, $CDCl_3$) δ (ppm) 8.69 (d, J=4.6 Hz, 1H, N**CH**), 7.78 (d, J=8.1 Hz, 1H, BrCC**CH**), 7.75 (d, J=4.8 Hz, 1H, BrC**CH**), 7.58 (t, J=8.2 Hz, 1H, Ar**H**), 7.14 (d, J=8.0 Hz, 1H, Ar**H**), 4.12 (s, 3H, **OMe**) ppm |
| **$^{13}$CNMR** | (101 MHz, $CDCl_3$) δ (ppm) 155.5 **(4),** 148.5 **(10),** 134.1 **(2),** 129.0 (7), 128.0 **(1),** 125.8 **(5,8),** 118.5 **(3),** 108.5 (**6**), 56.3 **(13)** ppm |
| **IR (neat)** | $v_{max}$ 1252 (s), 1085 (s) $cm^{-1}$ |
| **LCMS** | **(ESI$^+$)** *m/z* 238.0 [$M^{79}$Br+H]$^+$, 340.0 [$M^{81}$Br+H]$^+$ **(ESI$^+$)** *m/z* 260.0 [$M^{79}$Br+Na]$^+$, 262.0 [$M^{81}$Br+Na]$^+$ |
| **HRMS** | **(ESI$^+$)** *m/z calcd.* 237.9862 *meas.* 237.9865 [M+H]$^+$ |
| **Mpt** | 99 - 101 °C |

**Synthesis of N-(but-3-yn-1-yl)-8-methoxyquinolin-4-amine (16):**

3-Butyn-1-amine (0.86 mL, 10.5 mmol, 5 eq.) was mixed with 4-bromo-8-methoxyquinoline (500 mg, 2.1 mmol, 1 eq.) to form a paste. The paste was heated to 80 °C for 1 hour without stirring. The temperature was then raised to 140 °C and the reaction was stirred overnight. The reaction was cooled to room temperature and NaOH (1 M, 2 mL) was added. The organic product was basified with 10% aqueous NaOH and was extracted ($CH_2Cl_2$). The organic layers were combined, washed, dried ($MgSO_4$), filtered and concentrated *in vacuo.* The crude product was purified by column chromatography ($Al_2O_3$, 10% MeOH in EtOAc), affording N-(but-3-yn-1-yl)-8-methoxyquinolin-4-amine (250 mg, 2.17 mmol, 53%).
*Ref:* Austin et al., JACS, 1489-1497 (1958*)*

| | |
|---|---|
| **TLC $R_f$** | 0.73 (MeOH/ EtOAc 1:9, $Al_2O_3$) |
| **$^1$HNMR** | (400MHz, DMSO-$d_6$) δ (ppm) 8.36 (d, J=5.4 Hz, 1H, N**CH**), 7.73 (d, J=7.8 Hz, 1H, MeOCCHCH**CH**), 7.35 (t, J=8.1 Hz, 1H, MeOCCH**CH**), 7.10 (d, J=7.3 Hz, 1H, OMeC**CH**), 6.55 (d, J=5.4 Hz, 1H, NCH**CH**), 3.91 (s, 3H, **OMe**), 3.39-3.53 (m, 2H, **$CH_2$**$CH_2$CCH), 2.89 (t, J=2.6 Hz, 1H, $CH_2CH_2$C**CH**), 2.56 (td, J=7.1, 2.7 Hz, 2H, $CH_2$**$CH_2$**CCH) ppm |
| **$^{13}$CNMR** | (101 MHz, DMSO-$d_6$) δ (ppm) 155.2 **(3),** 149.9 **(9),** 148.8 **(8),** 139.6 (1), 124.3 **(4),** 119.6 **(6),** 113.3 **(2),** 108.5 **(5),** 99.1 **(10),** 82.5 **(16),** 72.6 **(17),** 55.8 **(12),** 41.5 **(14),** 18.0 **(15)** ppm |
| **IR (neat)** | $v_{max}$ 3279 (m), 2938 (m), 753 (s) $cm^{-1}$ |
| **LCMS** | **(ESI$^+$)** *m/z* 227.1 [M+H]$^+$ |
| **HRMS** | **(ESI$^+$)** *m/z calcd* 227.1179 *meas.* 227.1183 [M+H]$^+$ |
| **Mpt** | 154 - 155 °C |

**Synthesis of methyl 3-(benzyloxy)-6-(4-((8-methoxyquinolin-4-yl)amino)but-1-yn-1-yl) picolinate (17):**

To a degassed solution of methyl 3-(benzyloxy)-6-bromopyridine-2-carboxylate (391 mg, 1.22 mmol, 1.1 eq.) in THF/$Et_3$N (7 mL/3 mL), tetrakis(triphenylphosphine) palladium (127 mg, 0.11 mmol, 10 mol%) and copper iodide (48 mg, 0.22 mmol, 20 mol%) were added. After bubbling the reaction mixture with argon for 5 minutes at room temperature, a degassed solution of N-(but-3-yn-1-yl)-8-methoxyquinolin-4-amine (250 mg, 1.10 mmol, 1 eq.) in THF (3 mL) was added dropwise and the reaction mixture was stirred for 16 hours at room temperature. Upon completion, the reaction mixture was concentrated *in vacuo* and purified by column chromatography ($Al_2O_3$, 10% MeOH in EtOAc), affording methyl 3-(benzyloxy)-6-(4-((8-methoxyquinolin-4-yl)amino)but-1-yn-1-yl) picolinate (405 mg, 0.86 mmol, 78%).
*Ref:* Julien de Sousa Thesis, 2.49, 196 (2015*)*

| | |
|---|---|
| **TLC $R_f$** | 0.80 (MeOH/ EtOAc 1:9, $Al_2O_3$) |
| **$^1$HNMR** | (400MHz, DMSO-$d_6$) δ (ppm) 8.36 (br. d, J=5.1 Hz, 1H, N**CH**), 7.67-7.81 (m, 2H, NC**CH**), 7.57 (d, J=8.8, 1H, NCCH**CH**), 7.28- 7.47 (m, 7H, Ar**H**), 7.08 (br. d, J=7.6 Hz, 1H, MeOC**CH**), 6.60 (br. d, J=5.1 Hz, 2H, NCH**CH**) 5.26 (s, 2H, Ph**$CH_2$**), 3.90 (s, 3H, $CO_2$**$CH_3$**), 3.84 (s, 3H, **OMe**), 3.60-3.50 (m, 2H, NH**$CH_2$**$CH_2$), 2.82 (br. t, J=7.1 Hz, 2H, NH$CH_2$**$CH_2$**) ppm |
| **$^{13}$CNMR** | (101 MHz, DMSO-$d_6$) δ (ppm) 165.2 **(19),** 152.6 **(1),** 141.4 **(18),** 136.6 **(6),** 134.8 **(8),** 132.3 **(17),** 132.0 **(22),** 131.9 **(14),** 130.1 **(9),** 129.1 **(4),** 129.0 **(24),** 128.9 **(5),** 128.4 **(15),** 127.7 **(16),** 126.0 **(23),** 123.4 **(3),** 111.6 **(2),** 101.2 **(7),** 88.0 **(12),** 81.4 **(13),** 71.2 **(21),** 57.0 **(25),** 52.5 **(20),** 42.3 **(10),** 19.3 **(11)** ppm |
| **IR (neat)** | $v_{max}$ 2945 (m), 1722 (s), 695 (s) $cm^{-1}$ |

(continued)

| | |
|---|---|
| **LCMS** | **(ESI$^+$)** *m/z* 468.2 $[M+H]^+$ |
| **HRMS** | **(ESI$^+$)** *m/z calcd* 468.1931 *meas.* 468.1930 $[M+H]^+$ |
| **Mpt** | 181 - 183 °C |

**Synthesis of methyl 6-(4-((8-methoxyquinolin-4-yl)amino)butyl)-3-hydroxypicolinate (18):**

To a degassed solution of methyl 3-(benzyloxy)-6-(4-((8-methoxyquinolin-4-yl)amino)but-1-yn-1-yl) picolinate (200 mg, 0.43 mmol, 1 eq.) in dry MeOH (30 mL), was added Pearlman's catalyst (12 mg, 0.09 mmol, 20 mol%). After evacuating and flushing with hydrogen five times, the reaction mixture was stirred at room temperature, under a hydrogen atmosphere for 7 hours. Upon completion, the catalyst was removed by filtration through Celite and the solvent was removed *in vacuo,* affording methyl 6-(4-((8-methoxyquinolin-4-yl)amino)butyl)-3-hydroxypicolinate (163 mg, 0.42 mmol, 100%).
*Ref:* Julien de Sousa Thesis, 2.49, 196 (2015*)*

| | |
|---|---|
| **TLC $R_f$** | 0.00 (MeOH/ EtOAc 1:9, $Al_2O_3$) |
| **$^1$HNMR** | (400MHz, $CDCl_3$*)* δ (ppm) 8.50 (br. d, J=4.6 Hz, 1H, N**CH**), 7.23- 7.41 (m, 4H, Ar**H**), 7.00 (d, J=7.8, 1H, MeOC**CH**), 6.42 (br. d, J=5.9 Hz, 1H, NCH**CH**), 3.98 (s, 3H, $CO_2$**$CH_3$**), 3.97 (s, 3H, **OMe**), 3.42 (br. s, 2H, NH**$CH_2$**$CH_2CH_2$), 2.88 (t, J=7.2 Hz, 2H, $NHCH_2CH_2$**$CH_2$**), 1.79-1.92 (m, 4H, NH**$CH_2CH_2$**$CH_2$) ppm |
| **$^{13}$CNMR** | (101 MHz, $CDCl_3$) δ (ppm) 169.9 **(19),** 157.2 **(14),** 153.5 **(18),** 153.2 **(1),** 151.6 **(6),** 146.7 **(8),** 136.5 **(9),** 129.3 **(4),** 128.7 **(17),** 126.9 **(15),** 125.1 **(16),** 119.0 **(3),** 112.6 **(5),** 108.6 **(2),** 98.6 **(7),** 55.9 **(25),** 53.0 **(20),** 43.3 **(10),** 36.7 **(13),** 27.6 **(12),** 27.5 **(11)** ppm |
| **IR (neat)** | $v_{max}$ 2927 (m), 2359 (m), 1675 (s), 725 (s) $cm^{-1}$ |
| **LCMS** | **(ESI$^+$)** *m/z* 382.4 $[M+H]^+$ |
| **HRMS** | **(ESI$^+$)** *m/z calcd* 382.1775 *meas.* 382.1771 $[M+H]^+$ |
| **Mpt** | 156 - 159 °C |

**Synthesis of (E)-3-hydroxy-6-(4-((8-methoxyquinolin-4-yl)amino)butyl) picolinaldehyde oxime (K):**

To a degassed solution of methyl 6-(4-((8-methoxyquinolin-4-yl)amino)butyl)-3-hydroxypicolinate (50 mg, 0.13 mmol, 1 eq.) in dry $CH_2Cl_2$ (5 mL), was added 2,6-lutidine (42 mg, 0.05 mL, 0.39 mmol, 3 eq.) and TBSOTf (69 mg, 0.06 mL, 0.26 mmol, 2 eq.) successively and the reaction was stirred at room temperature for 5 hours under argon. The reaction mixture was concentrated *in vacuo.* To a solution of the residue in dry $CH_2Cl_2$ (5 mL) at -78 °C was added DIBAL-H (1 M in $CH_2Cl_2$) (56 mg, 0.40 mL, 0.40 mmol, 3 eq.) dropwise. The reaction was stirred at -78 °C for 1 hour and the reaction was quenched at -78 °C with MeOH (3 mL). The mixture was warmed to room temperature and the solvent was removed *in vacuo.* The aluminium salts were removed by filtration in $CH_2Cl_2$. The filtrate was concentrated *in vacuo* and the residue was dissolved in dry ethanol (7 mL). Hydroxylamine hydrochloride (18 mg, 0.26 mmol, 2 eq.) and sodium acetate (22 mg, 0.26 mmol, 2 eq.) were added and the solution was stirred at reflux for 16 hours. After concentration *in vacuo,* the crude product was washed with $CH_2Cl_2$. The crude product was purified by column chromatography on silica gel (10% MeOH in $CH_2Cl_2$) to afford (*E*)-3-hydroxy-6-(4-((8-methoxyquinolin-4-yl)amino)butyl) picolinaldehyde oxime (40 mg, 0.11 mmol, 83%).
*Ref:* Julien de Sousa Thesis, 2.49, 196 (2015*)*

| | |
|---|---|
| **TLC $R_f$** | 0.19 (MeOH/ $CH_2Cl_2$ 1:9, $SiO_2$) |
| **$^1$HNMR** | (400MHz, MeOD-$d_4$) δ (ppm) 8.16 (d, J=7.1 Hz, 1H, N**CH**), 8.10 (s, 2H, **$CH_2$**NOH), 7.78 (d, J=8.1 Hz, 1H, **CH**COMe), 7.54 (t, J=8.3 Hz, 1H, **CH**CHCOMe), 7.38 (d, J=7.6 Hz, 1H, **CH**CHCHCOMe), 7.07-7.21 (m, 2H, NC**CHCH**), 6.76 (d, J=7.3 Hz, 1H, NCH**CH**), 4.07 (s, 3H, **OMe**), 3.54 (t, J=7.1 Hz, 2H, NH**$CH_2$**$CH_2$), 2.74 (t, J=7.0 Hz, 2H, $NHCH_2CH_2CH_2$**$CH_2$**), 1.73-1.88 (m, 4H, $NHCH_2$**$CH_2CH_2$**$CH_2$) ppm |
| **$^{13}$CNMR** | (101 MHz, MeOD-$d_4$) δ (ppm) 157.4 **(1),** 154.2 **(19),** 153.9 **(6),** 152.8 **(14),** 151.1 **(18),** 142.1 **(8),** 136.4 **(17),** 128.5 **(9),** 126.1 **(15),** 125.7 **(16),** 123.5 **(3),** 120.3 **(2),** 114.6 **(5),** 113.3 **(4),** 99.7 (7), 57.4 **(20),** 44.5 **(10),** 37.3 **(13),** 28.3 **(12),** 28.5 **(11)** ppm |
| **IR (neat)** | $v_{max}$ 3451 (br), 1621 (s), 1043 (s) $cm^{-1}$ |
| **LCMS** | **(ESI$^+$)** *m/z* 355.3 $[M+H]^+$ |
| **HRMS** | **(ESI$^+$)** *m/z calcd* 367.1765 *meas.* 367.1765 $[M+H]^+$ |
| **Mpt** | 157 - 159 °C |

Example 4: Synthesis of Compound **H** according to the invention

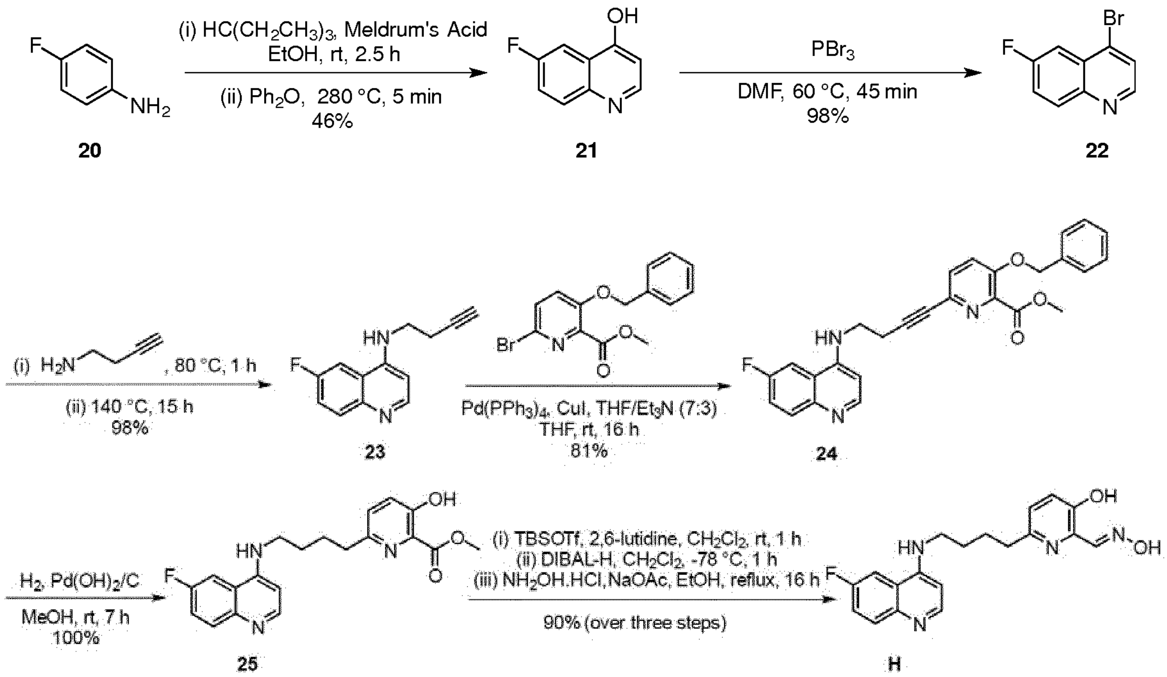


25 **H**

Scheme 3: Synthesis of Compound **H**

**Synthesis of 6-fluoroquinolin-4-ol (21):**

To a solution of p-fluoroaniline (3.03 mL, 31.5 mmol, 1 equiv) in ethanol (30 mL) were added Meldrum's acid (5.54 g, 38.4 mmol, 1.2 equiv) and triethyl orthoformate (12.4 mL, 74.5 mmol, 2.4 equiv). The mixture was heated to reflux for 2.5 h. The reaction was cooled to 0 °C and the solid was filtered and washed with cold ethanol. The dried solid was added portiowise over 5 mins to boiling diphenyl ether (100 mL). Reflux was maintained for an additional 3 mins. The reaction was stirred at room temperature for 30 mins before petroleum ether (25 mL) was added and the solid was filtered and dried. The crude product was purified by column chromatography on silica gel (EtOAc/MeOH 95:5), to afford 6-fluoroquinolin-4-ol (2.36 g, 14.5 mmol, 46%).

The analyses were consistent with the data reported in the literature.
*Ref:* WO2010/123995 (2010*)*

| | |
|---|---|
| **TLC $R_f$** | 0.41 (EtOAc/MeOH 95:5) |
| **$^1$HNMR** | 400MHz, DMSO-$d_6$) δ (ppm) 11.90 (s, 1H, **OH**), 7.94 (d, J=7.6, 1H, N**CH**), 7.73 (m, 1H, NC**CH**), 7.53-7.67 (m, 2H, FC**CH**, FC**CH**), 6.04 (d, J=7.3, 1H, OHC**CH**) ppm |
| **$^{13}$CNMR** | (101 MHz, DMSO-$d_6$) δ (ppm) 159.9 (7), 157.7 **(5),** 140.0 **(2),** 137.2 **(10),** 127.3 **(4),** 121.1 **(6),** 120.8 **(1),** 109.5 **(3),** 108.3 **(8)** ppm |
| **IR (neat)** | $v_{max}$ 2768 (br), 1594 (m) $cm^{-1}$ |
| **LCMS** | **(ESI$^+$)** *m/z* 164.1 $[M+H]^+$, 186.1 $[M+Na]^+$ |
| **HRMS** | **(ESI$^+$)** *m/z calcd.* 164.0506 *m/z meas.* 164.0509 $[M+H]^+$ |
| **Mpt** | 227 - 228 °C |

**Synthesis of AMI0016 - 4-bromo-6-fluoroquinoline (22):**

To a solution of *AMI0015* (2.30 g, 14.1 mmol, 1 equiv) in N,N-dimethylformamide (20 mL) at 60 °C was added phosphorus tribromide (1.52 mL, 16.2 mmol, 1.2 equiv). The mixture was cooled to 45 °C for 45 mins. After cooling to room temperature, the reaction was diluted with water (25 mL) and the pH was adjusted to ~ 10 with saturated aqueous

$NaHCO_3$. The resulting solid was washed in water, filtered and taken up in EtOAc and concentrated *in vacuo,* to afford 4-bromo-6-fluoroquinoline (3.12 g, 13.8 mmol, 98%).

The analyses were consistent with the data reported in the literature.
*Ref:* WO2010/152603 (2008*)*

| | |
|---|---|
| **TLC $R_f$** | 0.64 (EtOAc/MeOH 8:2) |
| **$^1$HNMR** | (400MHz, DMSO-$d_6$) δ (ppm) 8.73 (d, J=4.6 Hz, 1H, N**CH**), 8.18 (m, 1H, NC**CH**), 8.00 (d, J=4.6 Hz, 1H, BrC**CH**), 7.77-7.88 (m, 2H, FC**CH**, FC**CH**) ppm |
| **$^{13}$CNMR** | (101 MHz, DMSO-$d_6$) δ (ppm) 162.5 **(5),** 150.5 **(10),** 146.0 **(2),** 133.5 **(6),** 128.6 (7), 128.5 **(4),** 126.6 **(1),** 121.2 **(8),** 110.5 **(3)** ppm |
| **IR (neat)** | $v_{max}$ 1585 (m) $cm^{-1}$ |
| **LCMS** | **(ESI$^+$)** m/z 227.0 $[M^{79}Br+H]^+$, 229.0 $[M^{81}Br+H]^+$ |
| **HRMS** | **(ESI$^+$)** *m*/*z calcd.* 225.9662 *m*/*z meas.* 225.9962 $[M+H]^+$ |

**Synthesis of N-(but-3-yn-1-yl)-6-fluoroquinolin-4-amine (23):**

3-Butyn-1-amine (764 mg, 0.91 mL, 11.1 mmol, 5 eq.) was mixed with 4-bromo-6-fluoroquinoline (500 mg, 2.21 mmol, 1 eq.) to form a paste, which was heated to 80 °C for 1 hour without stirring, the temperature was raised to 140 °C and the reaction was left overnight with stirring. After cooling to room temperature, NaOH (1M, 2 mL) was added. The organic product was basified (10% NaOH) and extracted with $CH_2Cl_2$. The organic extracts were combined, dried ($MgSO_4$), filtered and concentrated *in vacuo.* The crude product was purified by column chromatography ($Al_2O_3$, 10% MeOH in EtOAc), affording N-(but-3-yn-1-yl)-6-fluoroquinolin-4-amine (465 mg, 2.17 mmol, 98%).
*Ref:* Austin et al., JACS, 1489-1497 (1958*)*

| | |
|---|---|
| **TLC $R_f$** | 0.75 (MeOH/ EtOAc 1:9, $Al_2O_3$) |
| **$^1$HNMR** | (400MHz, DMSO-$d_6$) δ (ppm) 8.41 (d, J=5.1 Hz, 1H, N**CH**), 7.89-8.08 (m, 1H, FCCHC**H**), 7.85 (dd, J=9.3, 5.9 Hz, 1H, FC**CH**CH), 7.53 (td, J=8.7, 2.8 Hz, 1H, FCC**HC**), 6.54 (d, J=5.4 Hz, 1H, NCH**CH**), 3.42-3.51 (m, 2H, $\mathbf{CH_2}CH_2CCH$), 2.90 (t, J=2.6 Hz, 1H, $CH_2CH_2C$**CH**), 2.56 (td, J=7.1, 2.7 Hz, 2H, $CH_2\mathbf{CH_2}CCH$) ppm |
| **$^{13}$CNMR** | (101 MHz, DMSO-$d_6$) δ (ppm) 150.5 **(4),** 150.2 **(7),** 149.1 **(9),** 140.0 **(1),** 124.6 **(2),** 119.9 **(3),** 113.6 **(6),** 108.8 **(5),** 99.4 **(8),** 72.9 **(16),** 56.0 **(13),** 41.8 **(11),** 18.3 **(12)** ppm |
| **IR (neat)** | $v_{max}$ 3387 (m), 2830 (m), 663 (s) $cm^{-1}$ |
| **LCMS** | (ESI$^+$) *m*/*z* 215.1 $[M+H]^+$ |
| **HRMS** | (ESI$^+$) *m*/*z calcd.* 215.0979 *m*/*z meas.* 215.0982 $[M+H]^+$ |
| **Mpt** | 148 - 150 °C |

**Synthesis of methyl 3-(benzyloxy)-6-(4-((6-fluoroquinolin-4-yl)amino)but-1-yn-1-yl) picolinate (24):**

To a degassed solution of methyl 3-(benzyloxy)-6-bromopyridine-2-carboxylate (496 mg, 1.54 mmol, 1.1 eq.) in THF/$Et_3N$ (7 mL/3 mL), tetrakis(triphenylphosphine) palladium (161 mg, 1.54 mmol, 10 mol%) and copper iodide (53 mg, 0.28 mmol, 20 mol%) were added. After bubbling the reaction mixture with argon for 5 minutes at room temperature, a degassed solution of N-(but-3-yn-1-yl)-6-fluoroquinolin-4-amine (330 mg, 1.4 mmol, 1 eq.) in THF (3 mL) was added dropwise and the reaction mixture was stirred for 16 hours at room temperature. Upon completion, the reaction mixture was concentrated *in vacuo* and purified by column chromatography ($SiO_2$, 10% MeOH in EtOAc), affording methyl 3-(benzyloxy)-6-(4-((6-fluoroquinolin-4-yl)amino)but-1-yn-1-yl) (515 mg, 1.13 mmol, 81%).
*Ref:* Julien de Sousa Thesis, 2.49, 196 (2015*)*

| | |
|---|---|
| **TLC $R_f$** | 0.20 (MeOH/EtOAc 1:9, $SiO_2$) |
| **$^1$HNMR** | (400MHz, DMSO-$d_6$) δ (ppm) 8.00-8.14 (m, 1H, N**CH**), 7.69 (d, J=8.8 Hz, 1H, NCCH**CH**CF), 7.57 (d, J=8.8, 1H, NC**CH**), 7.29-7.52 (m, 8H, Ar**H**), 6.61 (br. s, 1H, NCH**CH**), 5.26 (s, 2H, $Ph\mathbf{CH_2}$), 3.84 (s, 3H, $CO_2\mathbf{CH_3}$), 3.53-3.61 (m, 2H, $NH\mathbf{CH_2}CH_2$), 2.83 (t, J=7.0 Hz, 2H, $NHCH_2\mathbf{CH_2}$) ppm |
| **$^{13}$CNMR** | (101 MHz, DMSO-$d_6$) δ (ppm) 165.2 **(19),** 160.3 **(18),** 157.9 **(3),** 152.5 **(6),** 150.7 **(9),** 149.7 **(8),** 140.6 **(17),** 136.5 **(14),** 134.3 **(22),** 130.4 **(15),** 129.0 **(5),** 127.7 **(24),** 123.0 **(16),** 119.0 **(2),** 118.7 **(23),** 106.4 **(4),** 106.1 **(7),** **88.0 (12),** 80.8 **(13),** 70.4 **(21),** 52.8 **(20),** 41.7 **(10),** 19.0 **(11)** ppm |
| **IR (neat)** | $v_{max}$ 3255 (m), 2951 (m), 1725 (s), 721 (s) $cm^{-1}$ |

(continued)

| | |
|---|---|
| **LCMS** | **($ESI^+$)** *m/z* 456.2 $[M+H]^+$ |
| **HRMS** | **($ESI^+$)** *m/z calcd 456.1718 m/z meas.* 456.1720 $[M+H]^+$ |
| **Mpt** | 156 - 157 °C |

**Synthesis of methyl 6-(4-((6-fluoroquinolin-4-yl)amino)butyl)-3-hydroxypicolinate (25):**

To a degassed solution of methyl 3-(benzyloxy)-6-(4-((6-fluoroquinolin-4-yl)amino)but-1-yn-1-yl) picolinate (200 mg, 0.44 mmol, 1 eq.) in dry MeOH (40 mL), was added Pearlman's catalyst (12.3 mg, 0.087 mmol, 20 mol%). After evacuating and flushing with hydrogen five times, the reaction mixture was stirred at room temperature, under a hydrogen atmosphere for 7 hours. Upon completion, the catalyst was removed by filtration through Celite and the solvent was removed *in vacuo,* affording methyl 6-(4-((6-fluoroquinolin-4-yl)amino)butyl)-3-hydroxypicolinate (160 mg, 0.433 mmol, 99%).
*Ref:* Julien de Sousa Thesis, 2.49, 196 (2015*)*

| | |
|---|---|
| **TLC $R_f$** | 0.00 (EtOAc, $Al_2O_3$) |
| **$^1$HNMR** | (400MHz, DMSO-$d_6$) δ (ppm) 8.35 (d, J=5.1 Hz, 1H, N**CH**), 8.05 (dd, J=11.1, 2.8 Hz, 1H, NC**CH**), 7.81 (dd, J=9.3, 5.9 Hz, 2H, FC**CH**CH), 7.49 (td, J=8.7, 2.7 Hz, 1H, FC**CH**), 7.28-7.40 (m, 2H, NC**CHCH**COH), 6.44 (d, J=5.4 Hz, 1H, NCH**CH**), 3.85 (s, 3H, $CO_2$**$CH_3$**), 3.27-3.30 (m, 2H, HN**$CH_2$**$CH_2CH_2CH_2$), 2.67-2.78 (m, 2H, $NHCH_2CH_2CH_2$**$CH_2$**), 1.62-1.83 (m, 4H, $NHCH_2$**$CH_2CH_2$**$CH_2$) ppm |
| **$^{13}$CNMR** | (101 MHz, DMSO-$d_6$) δ (ppm) 169.2 **(19),** 160.6 **(3),** 158.2 **(14),** 157.2 **(18),** 153.2 **(6),** 150.9 **(8),** 144.5 **(9),** 130.0 **(17),** 129.0 **(15),** 127.1 **(1),** 124.7 **(16),** 118.6 **(2),** 105.2 **(5),** 105.0 **(4),** 98.0 **(7),** 51.8 **(20),** 42.3 **(10),** 35.9 **(13),** 29.3 **(11),** 27.3 **(12)** ppm |
| **IR (neat)** | $v_{max}$ 3240 (m), 2951 (m), 2860 (m), 2361 (s), 2342 (s), 1674 (s), 719 (s) $cm^{-1}$ |
| **LCMS** | **($ESI^+$)** *m/z* 370.2 $[M+H]^+$ |
| **HRMS** | **($ESI^+$)** *m/z calcd.* 370.1561 *m/z meas.* 370.1568 $[M+H]^+$ |
| **Mpt** | 155 - 157 °C |

**Synthesis of (E)-6-(4-((6-fluoroquinolin-4-yl)amino)butyl)-3-hydroxypicolinaldehyde oxime (H):**

To a degassed solution of methyl 6-(4-((6-fluoroquinolin-4-yl)amino)butyl)-3-hydroxypicolinate (100 mg, 0.27 mmol, 1 eq.) in dry $CH_2Cl_2$ (5 mL), was added 2,6-lutidine (87 mg, 0.09 mL, 0.81 mmol, 3 eq.) and TBSOTf (143 mg, 0.12 mL, 0.54 mmol, 2 eq.) successively and the reaction was stirred at room temperature for 5 hours under argon. The reaction mixture was concentrated *in vacuo.* To a solution of the residue in dry $CH_2Cl_2$ (5 mL) at -78 °C was added DIBAL-H (1 M in $CH_2Cl_2$) (115 mg, 0.81 mL, 0.81 mmol, 3 eq.) dropwise. The reaction was stirred at -78 °C for 1 hour and the reaction was quenched at -78 °C with MeOH (3 mL). The mixture was warmed to room temperature and the solvent was removed *in vacuo.* The aluminium salts were removed by filtration in $CH_2Cl_2$. The filtrate was concentrated *in vacuo* and the residue was dissolved in dry ethanol (7 mL). Hydroxylamine hydrochloride (37.6 mg, 0.54 mmol, 2 eq.) and sodium acetate (44.4 mg, 0.54 mmol, 2 eq.) were added and the solution was stirred at reflux for 16 hours. After concentration *in vacuo,* the crude product was washed with $CH_2Cl_2$. The crude product was purified by column chromatography ($SiO_2$, 10% MeOH in $CH_2Cl_2$) to afford (E)-6-(4-((6-fluoroquinolin-4-yl)amino)butyl)-3-hydroxypicolinaldehyde oxime (80 mg, 0.27 mmol, 90%).
*Ref:* Julien de Sousa Thesis, 2.49, 196 (2015*)*

| | |
|---|---|
| **TLC $R_f$** | 0.19 ($MeOH/CH_2Cl_2$ 1:9, $SiO_2$) |
| **$^1$HNMR** | (400MHz, MeOD-$d_4$) δ (ppm) 8.33 (d, J=6.6 Hz, 1H, N**CH**), 8.21 (s, 2H, **$CH_2$**NOH), 8.05 (dd, J=10.3, 2.7 Hz, 1H, NC**CH**), 7.88 (dd, J=9.2, 5.0 Hz, 1H, FC**CHC**), 7.67 (m, 1H, FC**CH**CH), 7.09-7.30 (m, 2H, NC**CHCH**), 6.71 (d, J=6.6 Hz, 1H, NCH**CH**), 3.47-3.56 (m, 2H, NH**$CH_2$**$CH_2$), 2.80 (t, J=7.1 Hz, 2H, $NHCH_2CH_2CH_2$**$CH_2$**), 1.75-1.96 (m, 4H, $NHCH_2$**$CH_2CH_2$**$CH_2$) ppm |
| **$^{13}$CNMR** | (101 MHz, MeOD-$d_4$) δ (ppm) 163.2 **(19),** 160.6 **(3),** 156.4 **(15),** 154.5 **(8),** 154.1 **(10),** 153.0 **(20),** 144.9 **(9),** 136.6 **(17),** 126.8 **(1),** 125.9 **(18),** 123.6 **(16),** 120.5 **(2),** 117.3 **(5),** 108.3 **(4), 99.6** (7), 44.7 **(11),** 37.5 **(14),** 28.6 **(13),** 28.5 **(12)** ppm |
| **IR (neat)** | $v_{max}$ 3673 (br), 1602 (s), 1033 (s) $cm^{-1}$ |
| **LCMS** | **($ESI^+$)** *m/z* 355.3 $[M+H]^+$ |
| **HRMS** | **($ESI^+$)** *m/z calcd.* 355.1561 *m/z meas.* 355.1561 $[M+H]^+$ |

(continued)

| | |
|---|---|
| **Mpt** | 157 - 159 °C |

Example 5: Reactivation of hAChE inhibited by OPNAs

*Materials and methods*

**$IC_{50}$ measurements.** Recombinant hAChE was produced and purified as previously described [Carletti et al 2008 JAmChemSoc 130(47):16011-20). Oximes were dissolved in MeOH to make 5 mM or 10 mM stock solution and further diluted in phosphate buffer (sodium phosphate 0.1 M, pH 7.4). Recombinant hAChE activity was measured spectrophotometrically (absorbance at 412 nm) in the presence of various concentrations of oximes in 1 mL Ellman's buffer (sodium phosphate 0.1 M, pH 7.4, 0.1% BSA, 0.5 mM DTNB, 25 °C). Measurements were performed at least in duplicate for each concentration tested. The concentration of oxime producing 50% of enzyme inhibition was determined by non-linear fitting using ProFit (Quantumsoft) using the standard $IC_{50}$ equation: $\%Activity=100*IC_{50}/(IC_{50}+[Ox])$.

**Inhibition of hAChE by OPNAs.** Recombinant hAChE was produced and purified as previously described (see reference: http://www.ncbi.nlm.nih.gov/pubmed/18975951) VX and tabun were from DGA maîtrise NRBC (Vert le Petit, France). Paraoxon-ethyl was purchased from Sigma-Aldrich. HI-6 was from Pharmacie Centrale des Armées (Orléans, France). All other chemicals including paraoxon were from Sigma. Stock solution of VX and tabun were 5 mM in isopropanol. The inhibition of 120 $\mu$M hAChE was carried out with a 5-fold excess of OPNAs and was performed in tris buffer (20 mM, pH 7.4, 0.1% BSA) at 25 °C. After a 20-minute incubation, inhibited hAChE was desalted on PD-10 column (GE Healthcare).

**Reactivation of hAChE inhibited by OPNAs.**

OPNA-inhibited hAChE was incubated at 37 °C with at least 4 or 5 concentrations of oxime in phosphate buffer (0.1 M, pH 7.4, 0.1% BSA). The final concentration of MeOH in the incubation mix was below 2% and had no influence on the enzyme stability. At time intervals ranging from 1 to 10 minutes depending on the reactivation rate, 10 $\mu$L aliquots of each solution containing the different concentrations of oxime were transferred to cuvettes containing 1 mM acetylthiocholine in 1 mL Ellman's buffer (phosphate 0.1 M, pH 7.4, 0.1% BSA, 0.5 mM DTNB, 25 °C) for measurement of hAChE activity.

The enzyme activity in the control remained constant during the experiment. The percentage of reactivated enzyme ($\%E_{react}$) was calculated as the ratio of the recovered enzyme activity and activity in the control. The apparent reactivation rate $k_{obs}$ for each oxime concentration, the dissociation constant $K_D$ of inhibited enzyme-oxime complex (E-POx) and the maximal reactivation rate constant $k_r$, were calculated by non-linear fit with ProFit (Quantumsoft) using the standard oxime concentration-dependent reactivation equation derived from the following

$$\text{E-P} + \text{Ox} \underset{}{\overset{K_D}{\rightleftharpoons}} \text{E-POx} \xrightarrow{k_r} \text{E} + \text{POx}$$

$$\%E_{react} = 100\ .\ (1-e^{k_{obs}\cdot t}) \qquad \text{and} \qquad k_{obs} = \frac{k_r[Ox]}{K_D + [Ox]}$$

*Results*

The ability of the following oximes to reactivate *in vitro* hAChE poisoned with various OPNAs was studied by spectrophotometry using Ellman's reagent and compared to known reactivators such as pralidoxime (also called 2-PAM), obidoxime (also called LuH-6), HLö-7, trimedoxime (also called TMB4) and HI-6:

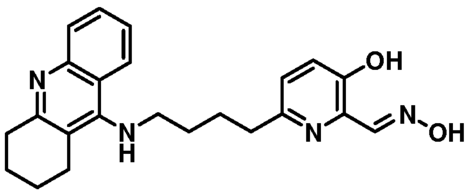

KM 297

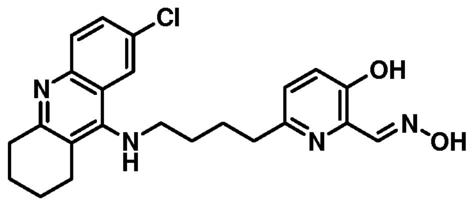

JDS 207

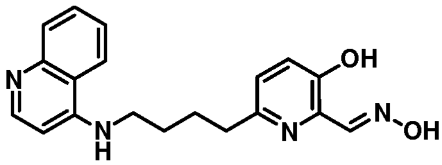

E

Compound **E** is as described in Example 1.

Compound KM 297 is described in Eur. J. Med. Chem. 2014, 6, 455-467 as compound **12.**

JDS 207 is described in WO 2015/075082 as compound **15.**

The results are provided in Table 1 below.

Table 1

| In vitro reactivation of AChE inhibited by various OPNAs | | | |
|---|---|---|---|
| VX | | | |
| **Test cpd.** | **$k_r$ (min$^{-1}$)** | **$K_D$ (uM)** | **$k_{r2}$ (mM$^{-1}$min$^{-1}$)*** |
| Pralidoxime | 0.06±0.01 | 215±75 | 0.28 |
| HI-6 | 0.44±0.15 | 50±26 | 9 |
| **KM 297** | **0.72±0.07** | **31±6** | **22** |
| **JDS 207** | **0.29±0.02** | **21.3±5.1** | **13.6** |
| **E** | **0.14±0.003** | **3.9±0.5** | **33.9** |
| | | | |
| Tabun | | | |
| **Test cpd.** | **$k_r$ (min$^{-1}$)** | **$K_D$ (μM)** | **$k_{r2}$(mM$^{-1}$min$^{-1}$)*** |
| Obidoxime | 0.04±0.006 | 250±110 | 0.16 |
| HLö-7 | 0.020±0.0007 | 106±15 | 0.2 |
| Trimedoxime | 0.085±0.005 | 145±25 | 0.7 |
| **KM 297** | **0.021±0.001** | **7.1±1.5** | **3** |
| **JDS 207** | **0.116 ± 0.006** | **10.4 ± 2.6** | **11.23** |
| **E** | **0.364±0.02** | **5.83±1.8** | **62.4** |
| | | | |
| Paraoxon | | | |
| **Test cpd.** | **$k_r$ (min$^{-1}$)** | **$K_D$ (μM)** | **$k_{r2}$ (mM$^{-1}$min$^{-1}$)*** |
| Obidoxime | 0.81 | 32.2 | 20 |
| HLö-7 | 0.63±0.04 | 210±31 | 3 |
| Trimedoxime | 0.34±0.02 | 47.8±6.9 | 17 |
| **KM 297** | **0.111±0.002** | **3.6±0.2** | **31** |

(continued)

| Paraoxon | | | |
|---|---|---|---|
| **Test cpd.** | **$k_r$ (min$^{-1}$)** | **$K_D$ (μM)** | **$k_{r2}$ (mM$^{-1}$min$^{-1}$)*** |
| **JDS 207** | **0.11±0.01** | **5.7±1.7** | **19.3** |
| **E** | **0.19 ± 0.01** | **9.3 ± 1.3** | **20.4** |
| | | | |
| Sarin | | | |
| **Test cpd.** | **$k_r$ (min$^{-1}$)** | **$K_D$ (μM)** | **$k_{r2}$ (mM$^{-1}$min$^{-1}$)*** |
| Obidoxime | 0.937 | 31.3 | 30.2 |
| HLö-7 | 0.849 | 24.2 | 35.0 |
| **KM 297** | **0.327 ± 0.009** | **20.4 ± 3** | **16** |
| **JDS 207** | **0.136 ± 0.007** | **11.5 ± 3** | **11.8** |
| **E** | **0.46 ± 0.016** | **24.97 ± 3** | **18.32** |
| * $k_{r2} = k_r/K_D$ | | | |

Significantly, the quinoline-based reactivator according to this invention outperformed most reactivators for VX- and tabun-inhibited hAChE. These data demonstrate in particular that it outclassed HI-6, which is known to be the most effective reactivator for VX poisoning to date, as well as trimedoxime, which is known to be the only reactivator moderately effective against tabun poisoning. Compound **E** further exhibited much higher *in vitro* reactivation potency of WFA-inhibited AChE compared to tacrine-based oximes (KM297 and JDS 207) due to a significantly improved affinity toward the inhibited enzyme ($K_D$). Moreover, this compound also effectively reactivated paraoxon- and sarin-inhibited AChE, which allows this compound to be applied against a broad spectrum of neurotoxic agents.

An additional experiment was performed to determine the first order reactivation rate constant, $k_{obs}$, of compound M according to this invention, compared with HI-6, at a concentration of 100 μM. The results obtained are summarized in Table 2 below.

Table 2

| In vitro reactivation of AChE inhibited by various OPNAs | | |
|---|---|---|
| **OPNA** | **$k_{obs}$ (min$^{-1}$)** | |
| | HI-6 | Cpd. M |
| VX | 0.37 | 0.66 |
| Tabun | 0.02 | 0.10 |
| Paraoxon | 0.01 | 0.12 |

As shown in this table, Compound **M** displays higher $k_{obs}$ than the commercial reactivator (HI-6) and this compound has thus the potential to be a good reactivator of OP-inhibited AchE.

Example 6: Inhibition of hAChE with compounds of the invention

As shown in Table 3, compound **E** was found to inhibit native hAChE.

Table 3

| Compound | $IC_{50}$ (μM) |
|---|---|
| Tacrine | 0.205±18 (human erythrocyte AChE)[a] |
| **E** | 1.4 ± 0.1 mM (hAChE) |

## Claims

1. Compound of formula (I)

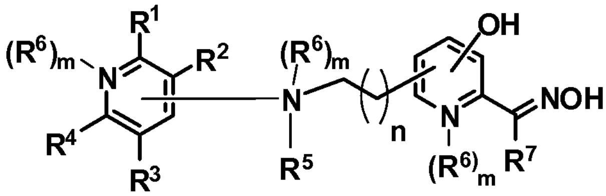

(I)

wherein:

n is 0, 1, 2, 3 or 4
m is 0 or 1,
$R^1$ and $R^2$ form together and with the carbon atoms linked thereto an aryl group, preferably a substituted or non-substituted phenyl group, thereby forming a quinoline structure,
$R^3$ and $R^4$ are independently selected from the group consisting of a hydrogen atom, a halogen atom, an alkyl group, an alkoxy group, $CF_3$ and an aryl group,
$R^5$ is selected from the group consisting of a hydrogen atom, an alkyl group and an acyl group,
$R^6$ is a hydrogen atom or an alkyl group, when present, and
$R^7$ is selected from the group consisting of a hydrogen atom, a $NH_2$ group and a $CF_3$ group.

2. Compound according to claim 1, wherein m is 0.

3. Compound according to claim 1, wherein m is 1 and $R^6$ = H.

4. Compound according to any one of claims 1 to 3, wherein n is 2, 3 or 4, preferably n is 3.

5. Compound according to any one of claims 1 to 4, wherein at least one, preferably at least two and more preferably each of $R^3$, $R^5$ and $R^7$ is a hydrogen atom.

6. Compound according to any one of claims 1 to 5, wherein $R^4$ is a hydrogen atom or an alkyl group such as a methyl group, preferably a hydrogen atom.

7. Compound according to any one of claims 1 to 6, wherein the pyridine ring in Formula (I) is substituted in position 6 by the quinoline-bearing group and/or in position 3 by the OH group.

8. Compound according to claim 1, which has the following formula (Ia):

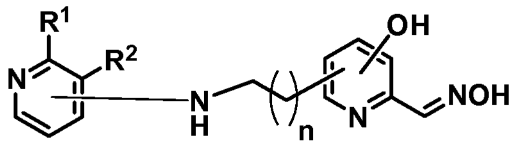

(Ia)

in which n is 2 or 3 and $R^1$ and $R^2$ form together and with the carbon atoms linked thereto a phenyl group which may be substituted or non substituted.

9. Compound according to claim 1, which is selected from the group consisting of:

- (E)-6-(4-((7-chloroquinolin-4-yl) amino)butyl)-3-hydroxypicolinaldehyde oxime
- (E)-6-(4-((7-fluoroquinolin-4-yl) amino)butyl)-3-hydroxypicolinaldehyde oxime

- (E)-6-(4-((7-(trifluoromethyl)quinolin-4-yl)amino)butyl)-3-hydroxy picolinaldehyde oxime
- (E)-6-(4-((6-methoxyquinolin-4-yl) amino)butyl)-3-hydroxypicolinaldehyde oxime
- (E)-6-(4-(quinolin-4-yl)amino)butyl)-3-hydroxy picolinaldehyde oxime
- (E)-6-(4-((6-chloro-8-methylquinolin-4-yl)amino)butyl)-3-hydroxy picolinaldehyde oxime
- (E)-6-(4-((6-fluoroquinolin-4-yl)amino)butyl)-3-hydroxy picolinaldehyde oxime
- (E)-6-(4-((8-fluoroquinolin-4-yl)amino)butyl)-3-hydroxy picolinaldehyde oxime
- (E)-6-(4-((6-(trifluoromethyl)quinolin-4-yl)amino)butyl)-3-hydroxy picolinaldehyde oxime
- (E)-6-(4-((8-methoxyquinolin-4-yl) amino)butyl)-3-hydroxypicolinaldehyde oxime

**10.** Pharmaceutical composition comprising at least one compound of formula (I) or (Ia) as defined in any one of claims 1 to 9 and at least one pharmaceutically acceptable support.

**11.** Compound according to any one of claims 1 to 9 for use as a medicine.

**12.** Compound according to any one of claims 1 to 9 for use in the treatment of a nervous and/or respiratory failure due to intoxication with at least one organophosphorous nerve agent.

**13.** Compound according to any one of claims 1 to 9 for use according to claim 12, wherein said organophosphorous nerve agent is selected from warfare agents such as *O*-ethyl S-[2-(diisopropylamino)ethyl] methylphosphonothioate (VX), tabun, sarin, cyclosarin and soman and pesticides such as paraoxon, parathion and tetraethyl pyrophosphate (TEPP).

**14.** Compound according to any one of claims 1 to 9, for use in the treatment of neurological diseases such as Alzheimer's disease.

**Patentansprüche**

**1.** Verbindung der Formel (I)

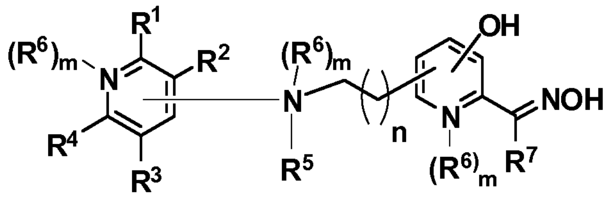

(I)

wobei:

n 0, 1, 2, 3 oder 4 ist,
m 0 oder 1 ist,
$R^1$ und $R^2$ zusammen und mit den Kohlenstoffatomen, an die sie gebunden sind, eine Arylgruppe, vorzugsweise eine substituierte oder nichtsubstituierte Phenylgruppe, bilden, wobei dadurch eine Chinolinstruktur gebildet wird,
$R^3$ und $R^4$ unabhängig voneinander aus der Gruppe ausgewählt sind, bestehend aus einem Wasserstoffatom, einem Halogenatom, einer Alkylgruppe, einer Alkoxygruppe, $CF_3$ und einer Arylgruppe,
$R^5$ aus der Gruppe ausgewählt ist, bestehend aus einem Wasserstoffatom, einer Alkylgruppe und einer Acylgruppe,
$R^6$, wenn vorhanden, ein Wasserstoffatom oder eine Alkylgruppe ist, und
$R^7$ aus der Gruppe ausgewählt ist, bestehend aus einem Wasserstoffatom, einer $NH_2$-Gruppe und einer $CF_3$-Gruppe.

**2.** Verbindung gemäß Anspruch 1, wobei m 0 ist.

**3.** Verbindung gemäß Anspruch 1, wobei m 1 und $R^6$ = H ist.

4. Verbindung gemäß einem der Ansprüche 1 bis 3, wobei n 2, 3 oder 4 ist, vorzugsweise n 3 ist.

5. Verbindung gemäß einem der Ansprüche 1 bis 4, wobei wenigstens ein, vorzugsweise wenigstens zwei oder mehr, vorzugsweise jeder Rest $R^3$, $R^5$ und $R^7$ ein Wasserstoffatom ist.

6. Verbindung gemäß einem der Ansprüche 1 bis 5, wobei $R^4$ ein Wasserstoffatom oder eine Alkylgruppe, wie eine Methylgruppe, vorzugsweise ein Wasserstoffatom ist.

7. Verbindung gemäß einem der Ansprüche 1 bis 6, wobei der Pyridinring in Formel (I) an Position 6 mit der Chinolin-tragenden Gruppe und/oder an Position 3 mit der OH-Gruppe substituiert ist.

8. Verbindung gemäß Anspruch 1, welche die folgende Formel (Ia) besitzt:

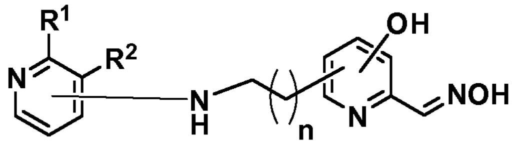

(Ia)

in der n 2 oder 3 ist und $R^1$ und $R^2$ zusammen und mit den Kohlenstoffatomen, an die sie gebunden sind, eine Phenylgruppe bilden, die substituiert oder nichtsubstituiert sein kann.

9. Verbindung gemäß Anspruch 1, die aus der Gruppe ausgewählt ist, bestehend aus:

- (E)-6-(4-((7-Chlorchinolin-4-yl) amino)butyl)-3-hydroxypicolinaldehydoxim
- (E)-6-(4-((7-Fluorchinolin-4-yl) amino)butyl)-3-hydroxypicolinaldehydoxim
- (E)-6-(4-((7-(Trifluormethyl)chinolin-4-yl)amino)butyl)-3-hydroxypicolinaldehydoxim
- (E)-6-(4-((6-Methoxychinolin-4-yl) amino)butyl)-3-hydroxypicolinaldehydoxim
- (E)-6-(4-(Chinolin-4-yl)amino)butyl)-3-hydroxypicolinaldehydoxim
- (E)-6-(4-((6-Chlor-8-methylchinolin-4-yl)amino)butyl)-3-hydroxypicolinaldehydoxim
- (E)-6-(4-((6-Fluorchinolin-4-yl)amino)butyl)-3-hydroxypicolinaldehydoxim
- (E)-6-(4-((8-Fluorchinolin-4-yl)amino)butyl)-3-hydroxypicolinaldehydoxim
- (E)-6-(4-((6-(Trifluormethyl)chinolin-4-yl)amino)butyl)-3-hydroxypicolinaldehydoxim
- (E)-6-(4-((8-Methoxychinolin-4-yl) amino)butyl)-3-hydroxypicolinaldehydoxim

10. Pharmazeutische Zusammensetzung umfassend wenigstens eine Verbindung der Formel (I) oder (Ia), wie in einem der Ansprüche 1 bis 9 definiert, und wenigstens einen pharmazeutisch verträglichen Träger.

11. Verbindung gemäß einem der Ansprüche 1 bis 9 zur Verwendung als ein Medikament.

12. Verbindung gemäß einem der Ansprüche 1 bis 9 zur Verwendung in der Behandlung eines Nervenversagens und/oder einer respiratorischen Insuffizienz aufgrund einer Intoxikation mit wenigstens einem Organophosphat-Nervengift.

13. Verbindung gemäß einem der Ansprüche 1 bis 9 zur Verwendung gemäß Anspruch 12, wobei besagtes Organophosphat-Nervengift aus Kampfstoffen, wie *O*-Ethyl-S-[2-(diisopropylamino)ethyl]methylphosphonothioat (VX), Tabun, Sarin, Cyclosarin und Soman, und Pestiziden, wie Paraoxon, Parathion und Tetraethylpyrophosphat (TEPP), ausgewählt ist.

14. Verbindung gemäß einem der Ansprüche 1 bis 9 zur Verwendung in der Behandlung neurologischer Erkrankungen wie Morbus Alzheimer.

## Revendications

1. Composé de formule (I)

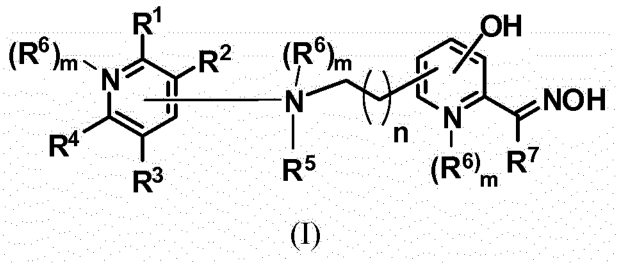

(I)

dans lequel :

n est 0,1, 2, 3 ou 4
m est 0 ou 1,
$R^1$ et $R^2$ forment ensemble et avec les atomes de carbone auxquels ils sont liés un groupe aryle, de préférence un groupe phényle substitué ou non substitué, formant ainsi une structure quinoléine,
$R^3$ et $R^4$ sont indépendamment choisis dans le groupe constitué par un atome d'hydrogène, un atome d'halogène, un groupe alkyle, un groupe alcoxy, $CF_3$ et un groupe aryle,
$R^5$ est choisi dans le groupe constitué par un atome d'hydrogène, un groupe alkyle et un groupe acyle,
$R^6$ est un atome d'hydrogène ou un groupe alkyle, quand il est présent, et
$R^7$ est choisi dans le groupe constitué par un atome d'hydrogène, un groupe $NH_2$ et un groupe $CF_3$,

**2.** Composé selon la revendication 1, dans lequel m est 0.

**3.** Composé selon la revendication 1, dans lequel m est 1 et $R^6$ = H.

**4.** Composé selon l'une quelconque des revendications 1 à 3, dans lequel n est 2, 3 ou 4, de préférence n est 3.

**5.** Composé selon l'une quelconque des revendications 1 à 4, dans lequel au moins un, de préférence au moins deux et plus préférablement chacun des $R^3$, $R^5$ et $R^7$ est un atome d'hydrogène.

**6.** Composé selon l'une quelconque des revendications 1 à 5, dans lequel $R^4$ est un atome d'hydrogène ou un groupe alkyle tel qu'un groupe méthyle, de préférence un atome d'hydrogène.

**7.** Composé selon l'une quelconque des revendications 1 à 6, dans lequel le cycle pyridine dans la Formule (I) est substitué en position 6 par le groupe portant la quinoléine et/ou en position 3 par le groupe OH.

**8.** Composé selon la revendication 1, qui répond à la formule (Ia) suivante :

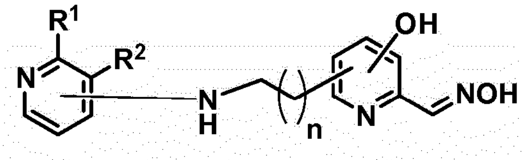

(Ia)

dans lequel n est 2 ou 3 et $R^1$ et $R^2$ forment ensemble et avec les atomes de carbone auxquels ils sont liés un groupe phényle qui peut être substitué ou non substitué.

**9.** Composé selon la revendication 1, qui est choisi dans le groupe constitué par :

- l'oxime de (E)-6-(4-((7-chloroquinoléin-4-yl)amino)butyl)-3-hydroxypicolinaldéhyde
- l'oxime de (E)-6-(4-((7-fluoroquinoléin-4-yl)amino)butyl)-3-hydroxypicolinaldéhyde
- l'oxime de (E)-6-(4-((7-trifluorométhyl)quinoléin-4-yl)amino)butyl)-3-hydroxy-picolinaldéhyde
- l'oxime de (E)-6-(4-((6-méthoxyquinoléin-4-yl)amino)butyl)-3-hydroxy-picolinaldéhyde
- l'oxime de (E)-6-(4-(quinoléin-4-yl)amino)butyl)-3-hydroxy-picolinaldéhyde
- l'oxime de (E)-6-(4-((6-chloro-8-méthylquinoléin-4-yl)amino)butyl)-3-hydroxy-picolinaldéhyde

- l'oxime de (E)-6-(4-((6-fluoroquinoléin-4-yl)amino)butyl)-3-hydroxy-picolinaldéhyde
- l'oxime de (E)-6-(4-((8-fluoroquinoléin-4-yl)amino)butyl)-3-hydroxy-picolinaldéhyde
- l'oxime de (E)-6-(4-((6-(trifluorométhyl)quinoléin-4-yl)amino)butyl)-3-hydroxy-picolinaldéhyde
- l'oxime de (E)-6-(4-((8-méthoxyquinoléin-4-yl)amino)butyl)-3-hydroxy-picolinaldéhyde

**10.** Composition pharmaceutique comprenant au moins un composé de formule (I) ou (Ia) tel que défini dans l'une quelconque des revendications 1 à 9 et au moins un support pharmaceutiquement acceptable.

**11.** Composé selon l'une quelconque des revendications 1 à 9 pour son utilisation comme médicament.

**12.** Composé selon l'une quelconque des revendications 1 à 9 pour son utilisation dans le traitement d'une défaillance du système nerveux et/ou d'une insuffisance respiratoire due à une intoxication par au moins un agent innervant organophosphoré.

**13.** Composé selon l'une quelconque des revendications 1 à 9 pour son utilisation selon la revendication 12 dans lequel ledit agent innervant organophosphoré est choisi parmi les agents de guerre tels que le S-[2-(diisopropylamino)éthyl]méthylphosphonothioate de *O*-éthyle (VX), le tabun, le sarin, le cyclosarin et le soman et les pesticides tels que le paraoxone, le parathion et le pyrophosphate de tétraéthyle (TEPP).

**14.** Composé selon l'une quelconque des revendications 1 à 9 pour son utilisation dans le traitement des maladies neurologiques telles que la maladie d'Alzheimer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015075082 A
- US 20110130429 A
- WO 2010123995 A
- WO 2010152603 A


### Non-patent literature cited in the description


- **MERCEY G. et al.** *Accounts of Chemical Research,* 2012, vol. 45 (5), 756-766
- **KLIACHYNA M. et al.** *European Journal of Medicinal Chemistry,* 2014, vol. 78, 455-467
- **SAINT-ANDRÉ et al.** *Tetrahedron,* 2011, vol. 67, 6352-6361
- **AUSTIN et al.** *JACS,* 1958, 1489-1497
- **JULIEN DE SOUSA.** *Thesis,* 2015, vol. 2.49, 196
- **CARLETTI et al.** *JAmChemSoc,* 2008, vol. 130 (47), 16011-20
- *Eur. J. Med. Chem.,* 2014, vol. 6, 455-467